# EUROPEAN PATENT APPLICATION

(11) **EP 3 974 442 A1**
(43) Date of publication of application: **30.03.2022**
(21) Application number: 20809910.1
(22) Date of filing: 25.05.2020
(51) Int. Cl.: C07K 14/435, C12N 5/10, C12N 1/15, C12N 1/19, C12N 1/21, C12N 15/12, C12P 21/02

(54) **MASS PRODUCTION SYSTEM OF RECOMBINANT BAGWORM SILK THREAD PROTEIN**

(30) Priority: 23.05.2019 JP 2019097154
(71) Applicant: KOWA COMPANY, LTD., Naka-ku Nagoya-shi Aichi 460-8625 (JP)
(72) Inventor: DOI, Takeshi, Tsukuba-shi, Ibaraki 305-0856 (JP); YOSHIDA, Hideo, Higashimurayama-shi, Tokyo 189-0022 (JP)
(74) Representative: Mewburn Ellis LLP
(86) International application number: PCT/JP2020/020457
(87) International publication number: WO 2020/235692

(57) **Abstract**

To develop a gene expression system whereby a recombinant bagworm fibroin H chain protein, which suffers from large suppression of the expression amount due to a gene expression controlling system in a microbial expression system, etc., can be expressed in a large amount even in a microbial expression system, etc. Provided is a gene expression reinforcement system for a recombinant bagworm fibroin H chain protein, said system comprising a gene encoding a modified recombinant bagworm fibroin H chain protein in which either the N-terminal domain or the C-terminal domain constituting the recombinant bagworm fibroin H chain protein has been partially or entirely deleted.

SEQUENCE LISTING

<110>  Kowa Company, Ltd.

<120>  Mass production system of recombinant bagworm silk proteins

<130>  PH-8445-PCT

<150>  2019-097154
<151>  2019-05-23

<160>  48

<170>  PatentIn version 3.5

<210>  1
<211>  110
<212>  PRT
<213>  Eumeta japonica

<400>  1

Met Ala Lys Ala Asp Trp Tyr Glu Asp Trp Lys Lys Asn Gln Gly Ser
1               5                   10                  15

Phe Arg Glu Thr Asp Leu Ala Asp Thr Asp Glu Tyr Gln Thr Asp Ser
            20                  25                  30

Asn Gly Thr Met Phe Glu Lys Lys Thr Thr Arg Lys Lys Phe Glu Lys
        35                  40                  45

Asp Gly Ser Thr Met Val Asn Ser Asp Ser Gly Glu Asp Lys Ile Val
    50                  55                  60

Arg Thr Phe Val Val Glu Thr Asp Ala Ser Gly His Glu Val Ile Tyr
65                  70                  75                  80

Glu Glu Asp Val Val Ile Lys Lys Val Pro Gly Lys Arg Lys Lys Val
                85                  90                  95

Ser Gln Ala Asn Ala Lys Ala Ser Ala Ile Ala Ala Ser Val
            100                 105                 110

<210>  2
<211>  32
<212>  PRT
<213>  Eumeta japonica

<400>  2

Phe Ala Ser Tyr Gly Arg Pro Gly Val Arg Gly Cys Gln Leu Ser Arg
1               5                   10                  15

Lys Tyr Leu Leu Val Lys Val Gly Leu Arg Ser Thr Cys Ser Asp Cys
            20                  25                  30

<210>  3
<211>  19
<212>  PRT
<213>  Eumeta japonica

<400>  3

Ala Ala Ala Ala Ala Ala Ala Ala Ala Gln Ala Ala Ala Ala Ala Ala
1               5                   10                  15

Ala Ala Ala

<210>  4
<211>  22
<212>  PRT
<213>  Eumeta japonica

<400>  4

Ala Ala Ala Ala Ala Ala Ala Ala Ala Glu Ala Ala Ala Ala Ala Ala
1               5                   10                  15

Ala Ala Ala Ala Ala Ala
            20

<210>  5
<211>  146
<212>  PRT
<213>  Eumeta japonica

<400>  5

Gly Ala Gly Val Gly Ala Ala Ser Val Ala Gly Ala Gly Thr Gly Ala
1               5                   10                  15

Gly Ser Gly Ala Ala Gly Gly Ala Gly Ala Gly Ala Asp Ala Gly Ala
            20                  25                  30

Ala Ala Ala Ala Ala Ala Ala Ala Gln Ala Ala Ala Ala Ala Ala Ala
        35                  40                  45

Ala Ala Gly Ser Gly Ala Gly Ala Gly Arg Val Gly Ala Tyr Gly Pro
    50                  55                  60

Tyr Gly Gly Leu Ala Ser Ala Gly Ala Gly Gly Ala Gly Gly Ala Gly
65                  70                  75                  80

Gly Ala Gly Gly Tyr Gly Gly Ala Ser Val Val Tyr Val Gly Gly Gly
                85                  90                  95

Gly Ala Gly Ala Gly Ala Gly Ser Gly Ala Gly Ala Gly Ser Gly Ala
            100                 105                 110

Gly Ala Gly Ala Gly Ser Gly Ala Gly Ala Gly Gly Ala Gly Ala Ala
        115                 120                 125

Ala Gly Ala Gly Ala Gly Ala Gly Ser Gly Ala Gly Ser Gly Ser Gly
    130                 135                 140

Ala Gly
145

<210>  6
<211>  153
<212>  PRT
<213>  Eumeta japonica

<400>  6

Ala Gly Ala Gly Ser Gly Ala Gly Ala Gly Ser Gly Ala Gly Ala Gly
1               5                   10                  15

Ala Gly Ala Gly Ser Gly Ala Ala Gly Gly Ala Gly Ala Gly Ala Gly
            20                  25                  30

Ala Gly Ala Gly Ala Ala Ala Ala Ala Ala Ala Ala Ala Glu Ala Ala
        35                  40                  45

Ala Ala Ala Ala Ala Ala Ala Ala Ala Ala Gly Ser Gly Ala Gly Ala
    50                  55                  60

Gly Gly Ala Gly Gly Tyr Gly Ala Gly Ala Gly Ala Gly Ala Gly Ala
65                  70                  75                  80

Gly Ala Gly Gly Ala Ala Gly Ala Gly Gly Ala Gly Gly Ala Gly Gly
                85                  90                  95

Ala Gly Gly Tyr Gly Gly Ala Ser Val Val Tyr Val Ser Ala Gly Gly
            100                 105                 110

Ala Gly Ala Gly Ala Gly Ser Gly Ala Gly Ala Gly Ser Gly Ala Gly
        115                 120                 125

Ala Gly Ala Gly Ser Gly Ala Gly Ala Gly Gly Ala Gly Ala Ala Ala
    130                 135                 140

Gly Ala Gly Ala Gly Ala Gly Ser Gly
145                 150

<210>  7
<211>  153
<212>  PRT
<213>  Eumeta japonica

<400>  7

Ala Gly Ser Gly Ser Gly Ala Gly Ala Gly Ala Gly Ser Gly Ala Val
1               5                   10                  15

Ala Gly Ser Gly Ala Gly Ser Gly Ala Ala Gly Gly Ala Gly Ala Gly
            20                  25                  30

Ala Gly Ala Gly Ala Ala Ala Ala Ala Ala Ala Ala Ala Glu Ala Ala
        35                  40                  45

Ala Ala Ala Ala Ala Ala Ala Ala Ala Ala Gly Ser Gly Ala Gly Ala
    50                  55                  60

Gly Gly Ala Gly Gly Tyr Gly Ala Gly Ala Gly Ala Gly Ala Gly Ala
65                  70                  75                  80

Gly Val Gly Gly Ala Gly Gly Ala Gly Gly Ala Gly Gly Ala Gly Gly
                85                  90                  95

Ala Gly Gly Tyr Gly Gly Ala Gly Val Val Tyr Val Ser Ala Gly Gly
            100                 105                 110

Ala Gly Ala Gly Ala Gly Ser Gly Ala Gly Ala Gly Ser Gly Ala Gly
        115                 120                 125

Ala Gly Ala Gly Ser Gly Ala Gly Ala Gly Gly Ala Gly Ala Ala Ala
    130                 135                 140

Gly Ala Gly Ala Gly Ala Gly Ser Gly
145                 150

<210>  8
<211>  159
<212>  PRT
<213>  Eumeta japonica

<400>  8

Ala Gly Ser Gly Ser Gly Ala Gly Ala Gly Ala Gly Ser Gly Ala Val
1               5                   10                  15

Ala Gly Ser Gly Ala Gly Ser Gly Ala Ala Gly Gly Ala Gly Ala Gly
            20                  25                  30

Ala Gly Ala Gly Ala Ala Ala Ala Ala Ala Ala Ala Ala Glu Ala Ala

35                  40                  45

Ala Ala Ala Ala Ala Ala Ala Ala Ala Ala Gly Ser Gly Ala Gly Ala
    50                  55                  60

Gly Gly Ala Gly Gly Tyr Gly Ala Gly Ala Gly Ala Gly Ala Gly Ala
65                  70                  75                  80

Gly Ala Gly Gly Ala Ser Gly Gly Tyr Gly Ser Tyr Gly Ser Gly Val
                85                  90                  95

Ala Ala Gly Ala Gly Ala Gly Ala Gly Val Gly Gly Ser Arg Gly Ala
            100                 105                 110

Gly Val Gly Ala Gly Val Gly Ala Gly Tyr Gly Ser Ala Leu Asn Ser
        115                 120                 125

Gly Ala Gly Ala Gly Ala Gly Ala Gly Ala Gly Ala Gly Gly Ala Ala
    130                 135                 140

Gly Ala Gly Ala Gly Ala Gly Ala Gly Ala Gly Ala Gly Ala Gly
145                 150                 155

<210>  9
<211>  161
<212>  PRT
<213>  Eumeta japonica

<400>  9

Ala Ala Ala Ala Ala Ala Ala Ala Ala Glu Ala Ala Ala Ala Ala Ala
1               5                   10                  15

Ala Ala Ala Ala Ala Ala Gly Ser Gly Ala Gly Ala Gly Gly Ala Gly
            20                  25                  30

Gly Tyr Gly Ala Gly Ala Gly Ala Gly Ala Gly Ala Gly Ala Gly Gly
        35                  40                  45

Ala Ala Gly Ala Gly Gly Ala Gly Gly Ala Gly Gly Ala Gly Gly Tyr
    50                  55                  60

Gly Gly Ala Ser Val Val Tyr Val Gly Gly Gly Gly Ala Gly Ala Gly
65                  70                  75                  80

Ala Gly Ser Gly Ala Gly Ala Gly Ser Gly Ala Gly Ala Gly Ala Gly
                85                  90                  95

Ser Gly Ala Gly Ala Gly Gly Ala Gly Ala Ala Ala Gly Ala Gly Ala

            100                 105                 110

Gly Ala Gly Ser Gly Ala Gly Ser Gly Ser Gly Ala Gly Ala Gly Ala
        115                 120                 125

Gly Ser Gly Ala Gly Ala Gly Ser Gly Ala Gly Ala Gly Ala Gly Ala
    130                 135                 140

Gly Ser Gly Ala Ala Gly Gly Ala Gly Ala Gly Ala Gly Ala Gly Ala
145                 150                 155                 160

Gly

<210> 10
<211> 159
<212> PRT
<213> Eumeta japonica

<400> 10

Ala Ala Ala Ala Ala Ala Ala Ala Ala Glu Ala Ala Ala Ala Ala Ala
1               5                   10                  15

Ala Ala Ala Ala Ala Ala Gly Ser Gly Ala Gly Ala Gly Gly Ala Gly
            20                  25                  30

Gly Tyr Gly Ala Gly Ala Gly Ala Gly Ala Gly Ala Gly Ala Gly Gly
        35                  40                  45

Ala Gly Gly Ala Gly Gly Ala Gly Gly Ala Gly Gly Ala Gly Gly Tyr
    50                  55                  60

Gly Gly Ala Gly Val Val Tyr Val Ser Ala Gly Gly Ala Gly Ala Gly
65                  70                  75                  80

Ala Gly Ser Gly Ala Gly Ala Gly Ser Gly Ala Gly Ala Gly Ala Gly
                85                  90                  95

Ser Gly Ala Gly Ala Gly Gly Ala Gly Ala Ala Ala Gly Ala Gly Ala
            100                 105                 110

Gly Ala Gly Ser Gly Ala Gly Ser Gly Ser Gly Ala Gly Ala Gly Ala
        115                 120                 125

Gly Ser Gly Ala Gly Ala Gly Ser Gly Ala Gly Ala Gly Ala Gly Ala
    130                 135                 140

Gly Ser Gly Ala Ala Gly Gly Ala Gly Ala Gly Ala Gly Ala Gly

145                 150                 155

<210>  11
<211>  153
<212>  PRT
<213>  Eumeta japonica

<400>  11

Ala Ala Ala Ala Ala Ala Ala Ala Ala Glu Ala Ala Ala Ala Ala Ala
1               5                   10                  15

Ala Ala Ala Ala Ala Ala Gly Ser Gly Ala Gly Ala Gly Gly Ala Gly
            20                  25                  30

Gly Tyr Gly Ala Gly Ala Gly Ala Gly Ala Gly Ala Gly Ala Gly Gly
        35                  40                  45

Ala Gly Gly Ala Gly Gly Ala Gly Gly Ala Gly Gly Ala Gly Gly Tyr
    50                  55                  60

Gly Gly Ala Gly Val Val Tyr Val Ser Ala Gly Gly Ala Gly Ala Gly
65                  70                  75                  80

Ala Gly Ser Gly Ala Gly Ala Gly Ser Gly Ala Gly Ala Gly Ala Gly
                85                  90                  95

Ser Gly Ala Gly Ala Gly Gly Ala Gly Ala Ala Ala Gly Ala Gly Ala
            100                 105                 110

Gly Ala Gly Ser Gly Ala Gly Ser Gly Ser Gly Ala Gly Ala Gly Ala
        115                 120                 125

Gly Ser Gly Ala Gly Ala Gly Ser Gly Ala Gly Ser Gly Ala Ala Gly
    130                 135                 140

Gly Ala Gly Ala Gly Ala Gly Ala Gly
145                 150

<210>  12
<211>  154
<212>  PRT
<213>  Eumeta japonica

<400>  12

Ala Ala Ala Ala Ala Ala Ala Ala Ala Glu Ala Ala Ala Ala Ala Ala
1               5                   10                  15

Ala Ala Ala Ala Ala Ala Gly Ser Gly Ala Gly Ala Gly Gly Ala Gly
            20                  25                  30

Gly Tyr Gly Ala Gly Ala Gly Ala Gly Ala Gly Ala Gly Ala Gly Gly
        35                  40                  45

Ala Gly Gly Ala Gly Gly Ala Gly Gly Ala Gly Gly Tyr Gly Gly Ala
    50                  55                  60

Ser Val Val Tyr Val Gly Ala Gly Gly Ala Gly Ala Gly Ala Gly Ser
65                  70                  75                  80

Gly Ala Gly Ala Gly Ser Gly Ala Gly Ala Gly Ala Gly Ser Gly Ala
                85                  90                  95

Gly Ala Gly Gly Ala Gly Ala Ala Ala Gly Ala Gly Ala Gly Ala Gly
            100                 105                 110

Ser Gly Ala Gly Ser Gly Ser Gly Ala Gly Ala Gly Ala Gly Ser Gly
        115                 120                 125

Ala Gly Ala Gly Ser Gly Ala Gly Ala Gly Ala Gly Ala Gly Ser Gly
    130                 135                 140

Ala Ala Gly Gly Ala Gly Ala Gly Ala Gly
145                 150

<210>  13
<211>  152
<212>  PRT
<213>  Eumeta japonica

<400>  13

Ala Ala Ala Ala Ala Ala Ala Ala Ala Glu Ala Ala Ala Ala Ala Ala
1               5                   10                  15

Ala Ala Ala Ala Ala Ala Gly Gly Ala Gly Gly Tyr Gly Pro Tyr Gly
            20                  25                  30

Gly Phe Ala Gly Ala Gly Ala Gly Ala Gly Gly Ala Gly Gly Ala Gly
        35                  40                  45

Gly Ala Gly Gly Ala Gly Gly Ala Gly Ser Thr Leu Ile Ile Val Asp
    50                  55                  60

Glu Gly Gly Tyr Gly Gly Ala Gly Gly Ala Gly Ser Gly Ala Gly Ser
65                  70                  75                  80

Gly Val Gly Ala Gly Ala Gly Ser Gly Ala Gly Ala Gly Gly Ala Gly
                85                  90                  95

Ala Ala Ala Gly Ala Gly Ala Gly Ala Gly Ser Gly Ala Gly Ser Gly
            100                 105                 110

Ser Gly Ala Gly Ala Gly Ala Gly Ser Gly Ala Gly Ala Gly Ser Gly
        115                 120                 125

Ala Gly Ala Gly Ala Gly Ala Gly Ser Gly Ala Ala Gly Gly Ala Gly
    130                 135                 140

Ala Gly Ala Gly Ala Gly Ala Gly
145                 150

<210>  14
<211>  1776
<212>  DNA
<213>  Eumeta japonica

<400>  14
atggcgaagg cggattggta tgaagattgg aaaaaaaacc aaggttcatt tagagaaaca      60

gacttagcag acactgacga atatcaaaca gatagtaatg gtacaatgtt tgaaaaaaaa     120

acaacaagaa aaaagttcga aaaagatgga agtactatgg taaacagtga ttccggagaa     180

gataaaattg tacgaacttt cgtcgtggaa actgacgcat caggacatga agttatttat     240

gaagaagatg tagtcattaa aaaagttcca ggtaaacgga agaaagtttc acaggcaaat     300

gctaaagcta gtgctatagc agccagtgta ggtgcgggcg ttggagcagc ttccgttgct     360

ggtgcaggaa caggagctgg ctcaggtgca gctggtggag caggagcagg tgctgacgct     420

ggcgctgcag ccgccgctgc ggcagcagca caagcagcag ccgcagctgc tgccgccgca     480

ggtagtggcg ctggtgctgg acgagtcgga gcttacggac cctacggagg tttagcaagc     540

gctggtgctg gtggtgctgg cggagccggt ggtgcaggtg gatacggtgg tgcaagtgtc     600

gtctacgtaa gtgctggagg agccggagca ggtgccggtt caggcgccgg tgctggatcg     660

ggtgccggag caggagctgg atcaggtgca ggagcaggtg gagcaggagc cgcagccgga     720

gctggtgctg gtgctggttc aggtgcagga tccggttcag gtgcgggcgc tggagcaggt     780

tccggagcag tagcaggttc cggtgctggt tcaggtgcag caggtggagc aggagctggc     840

gctggcgctg gcgctgcagc cgcagccgca gcagcagcag aagcggcagc cgcagctgcc     900

gccgccgctg ccgccgcagg tagtggagca ggcgctggag gagccggagg ctacggagcg     960

ggagctggag caggtgccgg agcaggtgtt ggcggcgctg gtggtgctgg tggtgctggc    1020

ggagccggtg gtgcaggtgg atacggtggt gcaggcgtcg tctacgtaag tgctggagga    1080

gccggagcag gtgccggttc aggcgccggt gctggatcgg gtgccggagc aggagctgga    1140

tcaggtgcag gagcaggtgg agcaggagcc gcagccggag ctggtgctgg tgctggttca    1200

ggtgcaggat ccggttcagg tgcgggcgct ggagcaggtt ccggagcagt agcaggttcc 1260

ggtgctggtt caggtgcagc aggtggagca ggagctggcg ctggcgctgg cgctgcagcc 1320

gcagccgcag cagcagcaga agcggcagcc gcagctgccg ccgccgctgc cgccgcaggt 1380

agtggagcag gcgctggagg agccggaggc tacggagcgg gagctggagc aggtgccggt 1440

gcaggtgctg gcggcgcatc tggaggctac ggttcttacg gatcgggagt tgcagcaggt 1500

gccggtgcag gagctggtgt tggtggtagc aggggagcag gtgttggcgc tggtgttggt 1560

gctggttatg gctccgcatt gaattcagga gccggtgccg gagcaggtgc tggtgctgga 1620

gctggtggtg ctgcaggagc tggtgctggt gcaggagcag gagctggcgc tggagctgga 1680

ttcgcttctt atggaagacc aggtgttcgt ggatgtcaac tgtctcgtaa ataccttttg 1740

gttaaagttg gtttaagatc cacatgctca gattgt 1776

<210> 15
<211> 592
<212> PRT
<213> Eumeta japonica

<400> 15

Met Ala Lys Ala Asp Trp Tyr Glu Asp Trp Lys Lys Asn Gln Gly Ser
1               5                   10                  15

Phe Arg Glu Thr Asp Leu Ala Asp Thr Asp Glu Tyr Gln Thr Asp Ser
            20                  25                  30

Asn Gly Thr Met Phe Glu Lys Lys Thr Thr Arg Lys Lys Phe Glu Lys
        35                  40                  45

Asp Gly Ser Thr Met Val Asn Ser Asp Ser Gly Glu Asp Lys Ile Val
    50                  55                  60

Arg Thr Phe Val Val Glu Thr Asp Ala Ser Gly His Glu Val Ile Tyr
65                  70                  75                  80

Glu Glu Asp Val Val Ile Lys Lys Val Pro Gly Lys Arg Lys Lys Val
                85                  90                  95

Ser Gln Ala Asn Ala Lys Ala Ser Ala Ile Ala Ala Ser Val Gly Ala
            100                 105                 110

Gly Val Gly Ala Ala Ser Val Ala Gly Ala Gly Thr Gly Ala Gly Ser
        115                 120                 125

Gly Ala Ala Gly Gly Ala Gly Ala Gly Ala Asp Ala Gly Ala Ala Ala
    130                 135                 140

Ala Ala Ala Ala Ala Ala Gln Ala Ala Ala Ala Ala Ala Ala Ala Ala
145                 150                 155                 160

Gly Ser Gly Ala Gly Ala Gly Arg Val Gly Ala Tyr Gly Pro Tyr Gly
                165                 170                 175

Gly Leu Ala Ser Ala Gly Ala Gly Gly Ala Gly Gly Ala Gly Gly Ala
            180                 185                 190

Gly Gly Tyr Gly Gly Ala Ser Val Val Tyr Val Ser Ala Gly Gly Ala
        195                 200                 205

Gly Ala Gly Ala Gly Ser Gly Ala Gly Ala Gly Ser Gly Ala Gly Ala
    210                 215                 220

Gly Ala Gly Ser Gly Ala Gly Ala Gly Gly Ala Gly Ala Ala Ala Gly
225                 230                 235                 240

Ala Gly Ala Gly Ala Gly Ser Gly Ala Gly Ser Gly Ser Gly Ala Gly
                245                 250                 255

Ala Gly Ala Gly Ser Gly Ala Val Ala Gly Ser Gly Ala Gly Ser Gly
            260                 265                 270

Ala Ala Gly Gly Ala Gly Ala Gly Ala Gly Ala Gly Ala Ala Ala Ala
        275                 280                 285

Ala Ala Ala Ala Ala Glu Ala Ala Ala Ala Ala Ala Ala Ala Ala Ala
    290                 295                 300

Ala Ala Gly Ser Gly Ala Gly Ala Gly Gly Ala Gly Gly Tyr Gly Ala
305                 310                 315                 320

Gly Ala Gly Ala Gly Ala Gly Ala Gly Val Gly Gly Ala Gly Gly Ala
                325                 330                 335

Gly Gly Ala Gly Gly Ala Gly Gly Ala Gly Gly Tyr Gly Gly Ala Gly
            340                 345                 350

Val Val Tyr Val Ser Ala Gly Gly Ala Gly Ala Gly Ala Gly Ser Gly
        355                 360                 365

Ala Gly Ala Gly Ser Gly Ala Gly Ala Gly Ala Gly Ser Gly Ala Gly
    370                 375                 380

Ala Gly Gly Ala Gly Ala Ala Ala Gly Ala Gly Ala Gly Ala Gly Ser

385                 390                 395                 400

Gly Ala Gly Ser Gly Ser Gly Ala Gly Ala Gly Ala Gly Ser Gly Ala
                405                 410                 415

Val Ala Gly Ser Gly Ala Gly Ser Gly Ala Ala Gly Gly Ala Gly Ala
            420                 425                 430

Gly Ala Gly Ala Gly Ala Ala Ala Ala Ala Ala Ala Ala Ala Glu Ala
        435                 440                 445

Ala Ala Ala Ala Ala Ala Ala Ala Ala Ala Ala Gly Ser Gly Ala Gly
    450                 455                 460

Ala Gly Gly Ala Gly Gly Tyr Gly Ala Gly Ala Gly Ala Gly Ala Gly
465                 470                 475                 480

Ala Gly Ala Gly Gly Ala Ser Gly Gly Tyr Gly Ser Tyr Gly Ser Gly
                485                 490                 495

Val Ala Ala Gly Ala Gly Ala Gly Ala Gly Val Gly Gly Ser Arg Gly
            500                 505                 510

Ala Gly Val Gly Ala Gly Val Gly Ala Gly Tyr Gly Ser Ala Leu Asn
        515                 520                 525

Ser Gly Ala Gly Ala Gly Ala Gly Ala Gly Ala Gly Ala Gly Gly Ala
    530                 535                 540

Ala Gly Ala Gly Ala Gly Ala Gly Ala Gly Ala Gly Ala Gly Ala Gly
545                 550                 555                 560

Phe Ala Ser Tyr Gly Arg Pro Gly Val Arg Gly Cys Gln Leu Ser Arg
                565                 570                 575

Lys Tyr Leu Leu Val Lys Val Gly Leu Arg Ser Thr Cys Ser Asp Cys
            580                 585                 590

<210>  16
<211>  1446
<212>  DNA
<213>  Eumeta japonica

<400>  16
atggcgggcg ttggagcagc ttccgttgct ggtgcaggaa caggagctgg ctcaggtgca        60

gctggtggag caggagcagg tgctgacgct ggcgctgcag ccgccgctgc ggcagcagca       120

caagcagcag ccgcagctgc tgccgccgca ggtagtggcg ctggtgctgg acgagtcgga       180

gcttacggac cctacggagg tttagcaagc gctggtgctg gtggtgctgg cggagccggt 240

ggtgcaggtg gatacggtgg tgcaagtgtc gtctacgtaa gtgctggagg agccggagca 300

ggtgccggtt caggcgccgg tgctggatcg ggtgccggag caggagctgg atcaggtgca 360

ggagcaggtg gagcaggagc cgcagccgga gctggtgctg gtgctggttc aggtgcagga 420

tccggttcag gtgcgggcgc tggagcaggt tccggagcag tagcaggttc cggtgctggt 480

tcaggtgcag caggtggagc aggagctggc gctggcgctg gcgctgcagc cgcagccgca 540

gcagcagcag aagcggcagc cgcagctgcc gccgccgctg ccgccgcagg tagtggagca 600

ggcgctggag gagccggagg ctacggagcg ggagctggag caggtgccgg agcaggtgtt 660

ggcggcgctg gtggtgctgg tggtgctggc ggagccggtg gtgcaggtgg atacggtggt 720

gcaggcgtcg tctacgtaag tgctggagga gccggagcag gtgccggttc aggcgccggt 780

gctggatcgg gtgccggagc aggagctgga tcaggtgcag gagcaggtgg agcaggagcc 840

gcagccggag ctggtgctgg tgctggttca ggtgcaggat ccggttcagg tgcgggcgct 900

ggagcaggtt ccggagcagt agcaggttcc ggtgctggtt caggtgcagc aggtggagca 960

ggagctggcg ctggcgctgg cgctgcagcc gcagccgcag cagcagcaga agcggcagcc 1020

gcagctgccg ccgccgctgc cgccgcaggt agtggagcag gcgctggagg agccggaggc 1080

tacggagcgg gagctggagc aggtgccggt gcaggtgctg gcggcgcatc tggaggctac 1140

ggttcttacg gatcgggagt tgcagcaggt gccggtgcag gagctggtgt tggtggtagc 1200

aggggagcag gtgttggcgc tggtgttggt gctggttatg gctccgcatt gaattcagga 1260

gccggtgccg gagcaggtgc tggtgctgga gctggtggtg ctgcaggagc tggtgctggt 1320

gcaggagcag gagctggcgc tggagctgga ttcgcttctt atggaagacc aggtgttcgt 1380

ggatgtcaac tgtctcgtaa ataccttttg gttaaagttg gtttaagatc cacatgctca 1440

gattgt 1446

<210> 17
<211> 482
<212> PRT
<213> Eumeta japonica

<400> 17

Met Ala Gly Val Gly Ala Ala Ser Val Ala Gly Ala Gly Thr Gly Ala
1               5                   10                  15

Gly Ser Gly Ala Ala Gly Gly Ala Gly Ala Gly Ala Asp Ala Gly Ala
            20                  25                  30

Ala Ala Ala Ala Ala Ala Ala Ala Gln Ala Ala Ala Ala Ala Ala Ala
        35                  40                  45

Ala Ala Gly Ser Gly Ala Gly Ala Gly Arg Val Gly Ala Tyr Gly Pro
        50                  55                  60

Tyr Gly Gly Leu Ala Ser Ala Gly Ala Gly Gly Ala Gly Gly Ala Gly
65                  70                  75                  80

Gly Ala Gly Gly Tyr Gly Gly Ala Ser Val Val Tyr Val Ser Ala Gly
                85                  90                  95

Gly Ala Gly Ala Gly Ala Gly Ser Gly Ala Gly Ala Gly Ser Gly Ala
            100                 105                 110

Gly Ala Gly Ala Gly Ser Gly Ala Gly Ala Gly Gly Ala Gly Ala Ala
        115                 120                 125

Ala Gly Ala Gly Ala Gly Ala Gly Ser Gly Ala Gly Ser Gly Ser Gly
    130                 135                 140

Ala Gly Ala Gly Ala Gly Ser Gly Ala Val Ala Gly Ser Gly Ala Gly
145                 150                 155                 160

Ser Gly Ala Ala Gly Gly Ala Gly Ala Gly Ala Gly Ala Gly Ala Ala
                165                 170                 175

Ala Ala Ala Ala Ala Ala Ala Glu Ala Ala Ala Ala Ala Ala Ala Ala
            180                 185                 190

Ala Ala Ala Ala Gly Ser Gly Ala Gly Ala Gly Gly Ala Gly Gly Tyr
        195                 200                 205

Gly Ala Gly Ala Gly Ala Gly Ala Gly Ala Gly Val Gly Gly Ala Gly
    210                 215                 220

Gly Ala Gly Gly Ala Gly Gly Ala Gly Gly Ala Gly Gly Tyr Gly Gly
225                 230                 235                 240

Ala Gly Val Val Tyr Val Ser Ala Gly Gly Ala Gly Ala Gly Ala Gly
                245                 250                 255

Ser Gly Ala Gly Ala Gly Ser Gly Ala Gly Ala Gly Ala Gly Ser Gly
            260                 265                 270

Ala Gly Ala Gly Gly Ala Gly Ala Ala Ala Gly Ala Gly Ala Gly Ala
        275                 280                 285

Gly Ser Gly Ala Gly Ser Gly Ser Gly Ala Gly Ala Gly Ala Gly Ser
    290                 295                 300

Gly Ala Val Ala Gly Ser Gly Ala Gly Ser Gly Ala Ala Gly Gly Ala
305                 310                 315                 320

Gly Ala Gly Ala Gly Ala Gly Ala Ala Ala Ala Ala Ala Ala Ala Ala
                325                 330                 335

Glu Ala Ala Ala Ala Ala Ala Ala Ala Ala Ala Ala Ala Gly Ser Gly
            340                 345                 350

Ala Gly Ala Gly Gly Ala Gly Gly Tyr Gly Ala Gly Ala Gly Ala Gly
        355                 360                 365

Ala Gly Ala Gly Ala Gly Gly Ala Ser Gly Gly Tyr Gly Ser Tyr Gly
    370                 375                 380

Ser Gly Val Ala Ala Gly Ala Gly Ala Gly Ala Gly Val Gly Gly Ser
385                 390                 395                 400

Arg Gly Ala Gly Val Gly Ala Gly Val Gly Ala Gly Tyr Gly Ser Ala
                405                 410                 415

Leu Asn Ser Gly Ala Gly Ala Gly Ala Gly Ala Gly Ala Gly Ala Gly
            420                 425                 430

Gly Ala Ala Gly Ala Gly Ala Gly Ala Gly Ala Gly Ala Gly Ala Gly
        435                 440                 445

Ala Gly Phe Ala Ser Tyr Gly Arg Pro Gly Val Arg Gly Cys Gln Leu
    450                 455                 460

Ser Arg Lys Tyr Leu Leu Val Lys Val Gly Leu Arg Ser Thr Cys Ser
465                 470                 475                 480

Asp Cys

<210>  18
<211>  1680
<212>  DNA
<213>  Eumeta japonica

<400>  18
atggcgaagg cggattggta tgaagattgg aaaaaaaacc aaggttcatt tagagaaaca      60

gacttagcag acactgacga atatcaaaca gatagtaatg gtacaatgtt tgaaaaaaaa     120

acaacaagaa aaaagttcga aaaagatgga agtactatgg taaacagtga ttccggagaa     180

gataaaattg tacgaacttt cgtcgtggaa actgacgcat caggacatga agttatttat     240

gaagaagatg tagtcattaa aaaagttcca ggtaaacgga agaaagtttc acaggcaaat 300

gctaaagcta gtgctatagc agccagtgta ggtgcgggcg ttggagcagc ttccgttgct 360

ggtgcaggaa caggagctgg ctcaggtgca gctggtggag caggagcagg tgctgacgct 420

ggcgctgcag ccgccgctgc ggcagcagca caagcagcag ccgcagctgc tgccgccgca 480

ggtagtggcg ctggtgctgg acgagtcgga gcttacggac cctacggagg tttagcaagc 540

gctggtgctg gtggtgctgg cggagccggt ggtgcaggtg gatacggtgg tgcaagtgtc 600

gtctacgtaa gtgctggagg agccggagca ggtgccggtt caggcgccgg tgctggatcg 660

ggtgccggag caggagctgg atcaggtgca ggagcaggtg gagcaggagc cgcagccgga 720

gctggtgctg gtgctggttc aggtgcagga tccggttcag gtgcgggcgc tggagcaggt 780

tccggagcag tagcaggttc cggtgctggt tcaggtgcag caggtggagc aggagctggc 840

gctggcgctg gcgctgcagc cgcagccgca gcagcagcag aagcggcagc cgcagctgcc 900

gccgccgctg ccgccgcagg tagtggagca ggcgctggag gagccggagg ctacggagcg 960

ggagctggag caggtgccgg agcaggtgtt ggcggcgctg gtggtgctgg tggtgctggc 1020

ggagccggtg gtgcaggtgg atacggtggt gcaggcgtcg tctacgtaag tgctggagga 1080

gccggagcag gtgccggttc aggcgccggt gctggatcgg gtgccggagc aggagctgga 1140

tcaggtgcag gagcaggtgg agcaggagcc gcagccggag ctggtgctgg tgctggttca 1200

ggtgcaggat ccggttcagg tgcgggcgct ggagcaggtt ccggagcagt agcaggttcc 1260

ggtgctggtt caggtgcagc aggtggagca ggagctggcg ctggcgctgg cgctgcagcc 1320

gcagccgcag cagcagcaga agcggcagcc gcagctgccg ccgccgctgc cgccgcaggt 1380

agtggagcag gcgctggagg agccggaggc tacggagcgg gagctggagc aggtgccggt 1440

gcaggtgctg gcggcgcatc tggaggctac ggttcttacg gatcgggagt tgcagcaggt 1500

gccggtgcag gagctggtgt tggtggtagc aggggagcag gtgttggcgc tggtgttggt 1560

gctggttatg gctccgcatt gaattcagga gccggtgccg gagcaggtgc tggtgctgga 1620

gctggtggtg ctgcaggagc tggtgctggt gcaggagcag gagctggcgc tggagctgga 1680

<210> 19
<211> 560
<212> PRT
<213> Eumeta japonica

<400> 19

Met Ala Lys Ala Asp Trp Tyr Glu Asp Trp Lys Lys Asn Gln Gly Ser
1               5                   10                  15

Phe Arg Glu Thr Asp Leu Ala Asp Thr Asp Glu Tyr Gln Thr Asp Ser
            20                  25                  30

Asn Gly Thr Met Phe Glu Lys Lys Thr Thr Arg Lys Lys Phe Glu Lys
        35                  40                  45

Asp Gly Ser Thr Met Val Asn Ser Asp Ser Gly Glu Asp Lys Ile Val
    50                  55                  60

Arg Thr Phe Val Val Glu Thr Asp Ala Ser Gly His Glu Val Ile Tyr
65                  70                  75                  80

Glu Glu Asp Val Val Ile Lys Lys Val Pro Gly Lys Arg Lys Lys Val
                85                  90                  95

Ser Gln Ala Asn Ala Lys Ala Ser Ala Ile Ala Ala Ser Val Gly Ala
            100                 105                 110

Gly Val Gly Ala Ala Ser Val Ala Gly Ala Gly Thr Gly Ala Gly Ser
        115                 120                 125

Gly Ala Ala Gly Gly Ala Gly Ala Gly Ala Asp Ala Gly Ala Ala Ala
    130                 135                 140

Ala Ala Ala Ala Ala Ala Gln Ala Ala Ala Ala Ala Ala Ala Ala Ala
145                 150                 155                 160

Gly Ser Gly Ala Gly Ala Gly Arg Val Gly Ala Tyr Gly Pro Tyr Gly
                165                 170                 175

Gly Leu Ala Ser Ala Gly Ala Gly Gly Ala Gly Gly Ala Gly Gly Ala
            180                 185                 190

Gly Gly Tyr Gly Gly Ala Ser Val Val Tyr Val Ser Ala Gly Gly Ala
        195                 200                 205

Gly Ala Gly Ala Gly Ser Gly Ala Gly Ala Gly Ser Gly Ala Gly Ala
    210                 215                 220

Gly Ala Gly Ser Gly Ala Gly Ala Gly Gly Ala Gly Ala Ala Ala Gly
225                 230                 235                 240

Ala Gly Ala Gly Ala Gly Ser Gly Ala Gly Ser Gly Ser Gly Ala Gly
                245                 250                 255

Ala Gly Ala Gly Ser Gly Ala Val Ala Gly Ser Gly Ala Gly Ser Gly
            260                 265                 270

Ala Ala Gly Gly Ala Gly Ala Gly Ala Gly Ala Gly Ala Ala Ala Ala
        275                 280                 285

Ala Ala Ala Ala Ala Glu Ala Ala Ala Ala Ala Ala Ala Ala Ala Ala
    290                 295                 300

Ala Ala Gly Ser Gly Ala Gly Ala Gly Gly Ala Gly Gly Tyr Gly Ala
305                 310                 315                 320

Gly Ala Gly Ala Gly Ala Gly Ala Gly Val Gly Gly Ala Gly Gly Ala
                325                 330                 335

Gly Gly Ala Gly Gly Ala Gly Gly Ala Gly Gly Tyr Gly Gly Ala Gly
            340                 345                 350

Val Val Tyr Val Ser Ala Gly Gly Ala Gly Ala Gly Ala Gly Ser Gly
        355                 360                 365

Ala Gly Ala Gly Ser Gly Ala Gly Ala Gly Ala Gly Ser Gly Ala Gly
    370                 375                 380

Ala Gly Gly Ala Gly Ala Ala Ala Gly Ala Gly Ala Gly Ala Gly Ser
385                 390                 395                 400

Gly Ala Gly Ser Gly Ser Gly Ala Gly Ala Gly Ala Gly Ser Gly Ala
                405                 410                 415

Val Ala Gly Ser Gly Ala Gly Ser Gly Ala Ala Gly Gly Ala Gly Ala
            420                 425                 430

Gly Ala Gly Ala Gly Ala Ala Ala Ala Ala Ala Ala Ala Ala Glu Ala
        435                 440                 445

Ala Ala Ala Ala Ala Ala Ala Ala Ala Ala Ala Gly Ser Gly Ala Gly
    450                 455                 460

Ala Gly Gly Ala Gly Gly Tyr Gly Ala Gly Ala Gly Ala Gly Ala Gly
465                 470                 475                 480

Ala Gly Ala Gly Gly Ala Ser Gly Gly Tyr Gly Ser Tyr Gly Ser Gly
                485                 490                 495

Val Ala Ala Gly Ala Gly Ala Gly Ala Gly Val Gly Gly Ser Arg Gly
            500                 505                 510

Ala Gly Val Gly Ala Gly Val Gly Ala Gly Tyr Gly Ser Ala Leu Asn
        515                 520                 525

Ser Gly Ala Gly Ala Gly Ala Gly Ala Gly Ala Gly Ala Gly Gly Ala

530                 535                 540

Ala Gly Ala Gly Ala Gly Ala Gly Ala Gly Ala Gly Ala Gly Ala Gly
545                 550                 555                 560

<210>  20
<211>  1350
<212>  DNA
<213>  Eumeta japonica

<400>  20
atggcgggcg ttggagcagc ttccgttgct ggtgcaggaa caggagctgg ctcaggtgca      60

gctggtggag caggagcagg tgctgacgct ggcgctgcag ccgccgctgc ggcagcagca     120

caagcagcag ccgcagctgc tgccgccgca ggtagtggcg ctggtgctgg acgagtcgga     180

gcttacggac cctacggagg tttagcaagc gctggtgctg gtggtgctgg cggagccggt     240

ggtgcaggtg gatacggtgg tgcaagtgtc gtctacgtaa gtgctggagg agccggagca     300

ggtgccggtt caggcgccgg tgctggatcg ggtgccggag caggagctgg atcaggtgca     360

ggagcaggtg gagcaggagc cgcagccgga gctggtgctg gtgctggttc aggtgcagga     420

tccggttcag gtgcgggcgc tggagcaggt tccggagcag tagcaggttc cggtgctggt     480

tcaggtgcag caggtggagc aggagctggc gctggcgctg gcgctgcagc cgcagccgca     540

gcagcagcag aagcggcagc cgcagctgcc gccgccgctg ccgccgcagg tagtggagca     600

ggcgctggag gagccggagg ctacggagcg ggagctggag caggtgccgg agcaggtgtt     660

ggcggcgctg gtggtgctgg tggtgctggc ggagccggtg gtgcaggtgg atacggtggt     720

gcaggcgtcg tctacgtaag tgctggagga gccggagcag gtgccggttc aggcgccggt     780

gctggatcgg gtgccggagc aggagctgga tcaggtgcag gagcaggtgg agcaggagcc     840

gcagccggag ctggtgctgg tgctggttca ggtgcaggat ccggttcagg tgcgggcgct     900

ggagcaggtt ccggagcagt agcaggttcc ggtgctggtt caggtgcagc aggtggagca     960

ggagctggcg ctggcgctgg cgctgcagcc gcagccgcag cagcagcaga agcggcagcc    1020

gcagctgccg ccgccgctgc cgccgcaggt agtggagcag gcgctggagg agccggaggc    1080

tacggagcgg gagctggagc aggtgccggt gcaggtgctg gcggcgcatc tggaggctac    1140

ggttcttacg gatcgggagt tgcagcaggt gccggtgcag gagctggtgt tggtggtagc    1200

aggggagcag gtgttggcgc tggtgttggt gctggttatg gctccgcatt gaattcagga    1260

gccggtgccg gagcaggtgc tggtgctgga gctggtggtg ctgcaggagc tggtgctggt    1320

gcaggagcag gagctggcgc tggagctgga                                     1350

<210>  21
<211>  450

<212>  PRT
<213>  Eumeta japonica

<400>  21

Met Ala Gly Val Gly Ala Ala Ser Val Ala Gly Ala Gly Thr Gly Ala
1               5                   10                  15

Gly Ser Gly Ala Ala Gly Gly Ala Gly Ala Gly Ala Asp Ala Gly Ala
            20                  25                  30

Ala Ala Ala Ala Ala Ala Ala Ala Gln Ala Ala Ala Ala Ala Ala Ala
        35                  40                  45

Ala Ala Gly Ser Gly Ala Gly Ala Gly Arg Val Gly Ala Tyr Gly Pro
    50                  55                  60

Tyr Gly Gly Leu Ala Ser Ala Gly Ala Gly Gly Ala Gly Gly Ala Gly
65                  70                  75                  80

Gly Ala Gly Gly Tyr Gly Gly Ala Ser Val Val Tyr Val Ser Ala Gly
                85                  90                  95

Gly Ala Gly Ala Gly Ala Gly Ser Gly Ala Gly Ala Gly Ser Gly Ala
            100                 105                 110

Gly Ala Gly Ala Gly Ser Gly Ala Gly Ala Gly Gly Ala Gly Ala Ala
        115                 120                 125

Ala Gly Ala Gly Ala Gly Ala Gly Ser Gly Ala Gly Ser Gly Ser Gly
    130                 135                 140

Ala Gly Ala Gly Ala Gly Ser Gly Ala Val Ala Gly Ser Gly Ala Gly
145                 150                 155                 160

Ser Gly Ala Ala Gly Gly Ala Gly Ala Gly Ala Gly Ala Gly Ala Ala
                165                 170                 175

Ala Ala Ala Ala Ala Ala Ala Glu Ala Ala Ala Ala Ala Ala Ala Ala
            180                 185                 190

Ala Ala Ala Ala Gly Ser Gly Ala Gly Ala Gly Gly Ala Gly Gly Tyr
        195                 200                 205

Gly Ala Gly Ala Gly Ala Gly Ala Gly Ala Gly Val Gly Gly Ala Gly
    210                 215                 220

Gly Ala Gly Gly Ala Gly Gly Ala Gly Gly Ala Gly Gly Tyr Gly Gly
225                 230                 235                 240

Ala Gly Val Val Tyr Val Ser Ala Gly Gly Ala Gly Ala Gly Ala Gly
                245                 250                 255

Ser Gly Ala Gly Ala Gly Ser Gly Ala Gly Ala Gly Ala Gly Ser Gly
            260                 265                 270

Ala Gly Ala Gly Gly Ala Gly Ala Ala Ala Gly Ala Gly Ala Gly Ala
        275                 280                 285

Gly Ser Gly Ala Gly Ser Gly Ser Gly Ala Gly Ala Gly Ala Gly Ser
    290                 295                 300

Gly Ala Val Ala Gly Ser Gly Ala Gly Ser Gly Ala Ala Gly Gly Ala
305                 310                 315                 320

Gly Ala Gly Ala Gly Ala Gly Ala Ala Ala Ala Ala Ala Ala Ala Ala
                325                 330                 335

Glu Ala Ala Ala Ala Ala Ala Ala Ala Ala Ala Ala Ala Gly Ser Gly
            340                 345                 350

Ala Gly Ala Gly Gly Ala Gly Gly Tyr Gly Ala Gly Ala Gly Ala Gly
        355                 360                 365

Ala Gly Ala Gly Ala Gly Gly Ala Ser Gly Gly Tyr Gly Ser Tyr Gly
    370                 375                 380

Ser Gly Val Ala Ala Gly Ala Gly Ala Gly Ala Gly Val Gly Gly Ser
385                 390                 395                 400

Arg Gly Ala Gly Val Gly Ala Gly Val Gly Ala Gly Tyr Gly Ser Ala
                405                 410                 415

Leu Asn Ser Gly Ala Gly Ala Gly Ala Gly Ala Gly Ala Gly Ala Gly
            420                 425                 430

Gly Ala Ala Gly Ala Gly Ala Gly Ala Gly Ala Gly Ala Gly Ala Gly
        435                 440                 445

Ala Gly
    450

<210>  22
<211>  2259
<212>  DNA

<213>  Eumeta japonica

<400>  22
atggcgaagg cggattggta tgaagattgg aaaaaaaacc aaggttcatt tagagaaaca      60

gacttagcag acactgacga atatcaaaca gatagtaatg gtacaatgtt tgaaaaaaaa     120

acaacaagaa aaaagttcga aaaagatgga agtactatgg taaacagtga ttccggagaa     180

gataaaattg tacgaacttt cgtcgtggaa actgacgcat caggacatga agttatttat     240

gaagaagatg tagtcattaa aaaagttcca ggtaaacgga agaaagtttc acaggcaaat     300

gctaaagcta gtgctatagc agccagtgta ggtgcgggcg ttggagcagc ttccgttgct     360

ggtgcaggaa caggagctgg ctcaggtgca gctggtggag caggagcagg tgctgacgct     420

ggcgctgcag ccgccgctgc ggcagcagca caagcagcag ccgcagctgc tgccgccgca     480

ggtagtggcg ctggtgctgg acgagtcgga gcttacggac cctacggagg tttagcaagc     540

gctggtgctg gtggtgctgg cggagccggt ggtgcaggtg gatacggtgg tgcaagtgtc     600

gtctacgtag gtggcggagg agccggagca ggtgccggtt caggcgccgg tgctggatcg     660

ggtgccggag caggagctgg atcaggtgca ggagcaggtg gagcaggagc cgcagccgga     720

gctggtgctg gtgctggttc aggtgcagga tccggttcag gtgcgggcgc tggagcaggt     780

tccggagcag gagcaggttc cggtgctggt gcaggagcag gagctggctc aggtgcagca     840

ggtggagcag gagcaggagc tggcgctggc gctggcgctg cagccgcagc cgcagcagca     900

gcagaagcgg cagccgcagc tgccgccgcc gctgccgccg caggtagtgg agcaggcgct     960

ggaggagccg gaggctacgg agcgggagct ggagcaggtg ccggtgcagg tgctggcggc    1020

gctgctggtg ctggtggtgc tggcggagcc ggtggtgcag gtggatacgg tggtgctagc    1080

gtcgtctacg taagtgctgg aggagccgga gcaggtgccg gttcaggcgc cggtgctgga    1140

tcgggtgccg gagcaggagc tggatcaggt gcaggagcag gtggagcagg agccgcagcc    1200

ggagctggtg ctggtgctgg ttcaggtgca ggatccggtt caggtgcggg cgctggagca    1260

ggttccggag cagtagcagg ttccggtgct ggttcaggtg cagcaggtgg agcaggagct    1320

ggcgctggcg ctggcgctgc agccgcagcc gcagcagcag cagaagcggc agccgcagct    1380

gccgccgccg ctgccgccgc aggtagtgga gcaggcgctg gaggagccgg aggctacgga    1440

gcgggagctg gagcaggtgc cggagcaggt gttggcggcg ctggtggtgc tggtggtgct    1500

ggcggagccg gtggtgcagg tggatacggt ggtgcaggcg tcgtctacgt aagtgctgga    1560

ggagccggag caggtgccgg ttcaggcgcc ggtgctggat cgggtgccgg agcaggagct    1620

ggatcaggtg caggagcagg tggagcagga gccgcagccg gagctggtgc tggtgctggt    1680

tcaggtgcag gatccggttc aggtgcgggc gctggagcag gttccggagc agtagcaggt    1740

tccggtgctg gttcaggtgc agcaggtgga gcaggagctg gcgctggcgc tggcgctgca    1800

gccgcagccg cagcagcagc agaagcggca gccgcagctg ccgccgccgc tgccgccgca 1860

ggtagtggag caggcgctgg aggagccgga ggctacggag cgggagctgg agcaggtgcc 1920

ggtgcaggtg ctggcggcgc atctggaggc tacggttctt acggatcggg agttgcagca 1980

ggtgccggtg caggagctgg tgttggtggt agcaggggag caggtgttgg cgctggtgtt 2040

ggtgctggtt atggctccgc attgaattca ggagccggtg ccggagcagg tgctggtgct 2100

ggagctggtg gtgctgcagg agctggtgct ggtgcaggag caggagctgg cgctggagct 2160

ggattcgctt cttatggaag accaggtgtt cgtggatgtc aactgtctcg taaatacctt 2220

ttggttaaag ttggtttaag atccacatgc tcagattgt 2259

<210> 23
<211> 753
<212> PRT
<213> Eumeta japonica

<400> 23

Met Ala Lys Ala Asp Trp Tyr Glu Asp Trp Lys Lys Asn Gln Gly Ser
1               5                   10                  15

Phe Arg Glu Thr Asp Leu Ala Asp Thr Asp Glu Tyr Gln Thr Asp Ser
            20                  25                  30

Asn Gly Thr Met Phe Glu Lys Lys Thr Thr Arg Lys Lys Phe Glu Lys
        35                  40                  45

Asp Gly Ser Thr Met Val Asn Ser Asp Ser Gly Glu Asp Lys Ile Val
    50                  55                  60

Arg Thr Phe Val Val Glu Thr Asp Ala Ser Gly His Glu Val Ile Tyr
65                  70                  75                  80

Glu Glu Asp Val Val Ile Lys Lys Val Pro Gly Lys Arg Lys Lys Val
                85                  90                  95

Ser Gln Ala Asn Ala Lys Ala Ser Ala Ile Ala Ala Ser Val Gly Ala
            100                 105                 110

Gly Val Gly Ala Ala Ser Val Ala Gly Ala Gly Thr Gly Ala Gly Ser
        115                 120                 125

Gly Ala Ala Gly Gly Ala Gly Ala Gly Ala Asp Ala Gly Ala Ala Ala
    130                 135                 140

Ala Ala Ala Ala Ala Ala Gln Ala Ala Ala Ala Ala Ala Ala Ala Ala
145                 150                 155                 160

Gly Ser Gly Ala Gly Ala Gly Arg Val Gly Ala Tyr Gly Pro Tyr Gly
                165                 170                 175

Gly Leu Ala Ser Ala Gly Ala Gly Gly Ala Gly Gly Ala Gly Gly Ala
            180                 185                 190

Gly Gly Tyr Gly Gly Ala Ser Val Val Tyr Val Gly Gly Gly Gly Ala
        195                 200                 205

Gly Ala Gly Ala Gly Ser Gly Ala Gly Ala Gly Ser Gly Ala Gly Ala
    210                 215                 220

Gly Ala Gly Ser Gly Ala Gly Ala Gly Gly Ala Gly Ala Ala Ala Gly
225                 230                 235                 240

Ala Gly Ala Gly Ala Gly Ser Gly Ala Gly Ser Gly Ser Gly Ala Gly
                245                 250                 255

Ala Gly Ala Gly Ser Gly Ala Gly Ala Gly Ser Gly Ala Gly Ala Gly
            260                 265                 270

Ala Gly Ala Gly Ser Gly Ala Ala Gly Gly Ala Gly Ala Gly Ala Gly
        275                 280                 285

Ala Gly Ala Gly Ala Ala Ala Ala Ala Ala Ala Ala Ala Glu Ala Ala
    290                 295                 300

Ala Ala Ala Ala Ala Ala Ala Ala Ala Ala Gly Ser Gly Ala Gly Ala
305                 310                 315                 320

Gly Gly Ala Gly Gly Tyr Gly Ala Gly Ala Gly Ala Gly Ala Gly Ala
                325                 330                 335

Gly Ala Gly Gly Ala Ala Gly Ala Gly Gly Ala Gly Gly Ala Gly Gly
            340                 345                 350

Ala Gly Gly Tyr Gly Gly Ala Ser Val Val Tyr Val Ser Ala Gly Gly
        355                 360                 365

Ala Gly Ala Gly Ala Gly Ser Gly Ala Gly Ala Gly Ser Gly Ala Gly
    370                 375                 380

Ala Gly Ala Gly Ser Gly Ala Gly Ala Gly Gly Ala Gly Ala Ala Ala
385                 390                 395                 400

Gly Ala Gly Ala Gly Ala Gly Ser Gly Ala Gly Ser Gly Ser Gly Ala
                405                 410                 415

Gly Ala Gly Ala Gly Ser Gly Ala Val Ala Gly Ser Gly Ala Gly Ser
            420                 425                 430

Gly Ala Ala Gly Gly Ala Gly Ala Gly Ala Gly Ala Gly Ala Ala Ala
        435                 440                 445

Ala Ala Ala Ala Ala Ala Glu Ala Ala Ala Ala Ala Ala Ala Ala Ala
    450                 455                 460

Ala Ala Ala Gly Ser Gly Ala Gly Ala Gly Gly Ala Gly Gly Tyr Gly
465                 470                 475                 480

Ala Gly Ala Gly Ala Gly Ala Gly Ala Gly Val Gly Gly Ala Gly Gly
                485                 490                 495

Ala Gly Gly Ala Gly Gly Ala Gly Gly Ala Gly Gly Tyr Gly Gly Ala
            500                 505                 510

Gly Val Val Tyr Val Ser Ala Gly Gly Ala Gly Ala Gly Ala Gly Ser
        515                 520                 525

Gly Ala Gly Ala Gly Ser Gly Ala Gly Ala Gly Ala Gly Ser Gly Ala
    530                 535                 540

Gly Ala Gly Gly Ala Gly Ala Ala Ala Gly Ala Gly Ala Gly Ala Gly
545                 550                 555                 560

Ser Gly Ala Gly Ser Gly Ser Gly Ala Gly Ala Gly Ala Gly Ser Gly
                565                 570                 575

Ala Val Ala Gly Ser Gly Ala Gly Ser Gly Ala Ala Gly Gly Ala Gly
            580                 585                 590

Ala Gly Ala Gly Ala Gly Ala Ala Ala Ala Ala Ala Ala Ala Ala Glu
        595                 600                 605

Ala Ala Ala Ala Ala Ala Ala Ala Ala Ala Ala Ala Gly Ser Gly Ala
    610                 615                 620

Gly Ala Gly Gly Ala Gly Gly Tyr Gly Ala Gly Ala Gly Ala Gly Ala
625                 630                 635                 640

Gly Ala Gly Ala Gly Gly Ala Ser Gly Gly Tyr Gly Ser Tyr Gly Ser
                645                 650                 655

Gly Val Ala Ala Gly Ala Gly Ala Gly Ala Gly Val Gly Gly Ser Arg

660                 665                 670

Gly Ala Gly Val Gly Ala Gly Val Gly Ala Gly Tyr Gly Ser Ala Leu
        675                 680                 685

Asn Ser Gly Ala Gly Ala Gly Ala Gly Ala Gly Ala Gly Ala Gly Gly
    690                 695                 700

Ala Ala Gly Ala Gly Ala Gly Ala Gly Ala Gly Ala Gly Ala Gly Ala
705                 710                 715                 720

Gly Phe Ala Ser Tyr Gly Arg Pro Gly Val Arg Gly Cys Gln Leu Ser
                725                 730                 735

Arg Lys Tyr Leu Leu Val Lys Val Gly Leu Arg Ser Thr Cys Ser Asp
            740                 745                 750

Cys

<210>  24
<211>  1929
<212>  DNA
<213>  Eumeta japonica

<400>  24
atggcgggcg ttggagcagc ttccgttgct ggtgcaggaa caggagctgg ctcaggtgca      60

gctggtggag caggagcagg tgctgacgct ggcgctgcag ccgccgctgc ggcagcagca     120

caagcagcag ccgcagctgc tgccgccgca ggtagtggcg ctggtgctgg acgagtcgga     180

gcttacggac cctacggagg tttagcaagc gctggtgctg gtggtgctgg cggagccggt     240

ggtgcaggtg gatacggtgg tgcaagtgtc gtctacgtag gtggcggagg agccggagca     300

ggtgccggtt caggcgccgg tgctggatcg ggtgccggag caggagctgg atcaggtgca     360

ggagcaggtg gagcaggagc cgcagccgga gctggtgctg gtgctggttc aggtgcagga     420

tccggttcag gtgcgggcgc tggagcaggt tccggagcag gagcaggttc cggtgctggt     480

gcaggagcag gagctggctc aggtgcagca ggtggagcag gagcaggagc tggcgctggc     540

gctggcgctg cagccgcagc cgcagcagca gcagaagcgg cagccgcagc tgccgccgcc     600

gctgccgccg caggtagtgg agcaggcgct ggaggagccg gaggctacgg agcgggagct     660

ggagcaggtg ccggtgcagg tgctggcggc gctgctggtg ctggtggtgc tggcggagcc     720

ggtggtgcag gtggatacgg tggtgctagc gtcgtctacg taagtgctgg aggagccgga     780

gcaggtgccg gttcaggcgc cggtgctgga tcgggtgccg gagcaggagc tggatcaggt     840

gcaggagcag gtggagcagg agccgcagcc ggagctggtg ctggtgctgg ttcaggtgca     900

ggatccggtt caggtgcggg cgctggagca ggttccggag cagtagcagg ttccggtgct 960

ggttcaggtg cagcaggtgg agcaggagct ggcgctggcg ctggcgctgc agccgcagcc 1020

gcagcagcag cagaagcggc agccgcagct gccgccgccg ctgccgccgc aggtagtgga 1080

gcaggcgctg gaggagccgg aggctacgga gcgggagctg gagcaggtgc cggagcaggt 1140

gttggcggcg ctggtggtgc tggtggtgct ggcggagccg gtggtgcagg tggatacggt 1200

ggtgcaggcg tcgtctacgt aagtgctgga ggagccggag caggtgccgg ttcaggcgcc 1260

ggtgctggat cgggtgccgg agcaggagct ggatcaggtg caggagcagg tggagcagga 1320

gccgcagccg gagctggtgc tggtgctggt tcaggtgcag gatccggttc aggtgcgggc 1380

gctggagcag gttccggagc agtagcaggt tccggtgctg gttcaggtgc agcaggtgga 1440

gcaggagctg gcgctggcgc tggcgctgca gccgcagccg cagcagcagc agaagcggca 1500

gccgcagctg ccgccgccgc tgccgccgca ggtagtggag caggcgctgg aggagccgga 1560

ggctacggag cgggagctgg agcaggtgcc ggtgcaggtg ctggcggcgc atctggaggc 1620

tacggttctt acggatcggg agttgcagca ggtgccggtg caggagctgg tgttggtggt 1680

agcaggggag caggtgttgg cgctggtgtt ggtgctggtt atggctccgc attgaattca 1740

ggagccggtg ccggagcagg tgctggtgct ggagctggtg gtgctgcagg agctggtgct 1800

ggtgcaggag caggagctgg cgctggagct ggattcgctt cttatggaag accaggtgtt 1860

cgtggatgtc aactgtctcg taaatacctt ttggttaaag ttggtttaag atccacatgc 1920

tcagattgt 1929

<210> 25
<211> 643
<212> PRT
<213> Eumeta japonica

<400> 25

Met Ala Gly Val Gly Ala Ala Ser Val Ala Gly Ala Gly Thr Gly Ala
1               5                   10                  15

Gly Ser Gly Ala Ala Gly Gly Ala Gly Ala Gly Ala Asp Ala Gly Ala
            20                  25                  30

Ala Ala Ala Ala Ala Ala Ala Ala Gln Ala Ala Ala Ala Ala Ala Ala
        35                  40                  45

Ala Ala Gly Ser Gly Ala Gly Ala Gly Arg Val Gly Ala Tyr Gly Pro
    50                  55                  60

Tyr Gly Gly Leu Ala Ser Ala Gly Ala Gly Gly Ala Gly Gly Ala Gly
65                  70                  75                  80

Gly Ala Gly Gly Tyr Gly Gly Ala Ser Val Val Tyr Val Gly Gly Gly
                85                  90                  95

Gly Ala Gly Ala Gly Ala Gly Ser Gly Ala Gly Ala Gly Ser Gly Ala
            100                 105                 110

Gly Ala Gly Ala Gly Ser Gly Ala Gly Ala Gly Gly Ala Gly Ala Ala
        115                 120                 125

Ala Gly Ala Gly Ala Gly Ala Gly Ser Gly Ala Gly Ser Gly Ser Gly
    130                 135                 140

Ala Gly Ala Gly Ala Gly Ser Gly Ala Gly Ala Gly Ser Gly Ala Gly
145                 150                 155                 160

Ala Gly Ala Gly Ala Gly Ser Gly Ala Ala Gly Gly Ala Gly Ala Gly
                165                 170                 175

Ala Gly Ala Gly Ala Gly Ala Ala Ala Ala Ala Ala Ala Ala Ala Glu
            180                 185                 190

Ala Ala Ala Ala Ala Ala Ala Ala Ala Ala Ala Ala Gly Ser Gly Ala
        195                 200                 205

Gly Ala Gly Gly Ala Gly Gly Tyr Gly Ala Gly Ala Gly Ala Gly Ala
    210                 215                 220

Gly Ala Gly Ala Gly Gly Ala Ala Gly Ala Gly Gly Ala Gly Gly Ala
225                 230                 235                 240

Gly Gly Ala Gly Gly Tyr Gly Gly Ala Ser Val Val Tyr Val Ser Ala
                245                 250                 255

Gly Gly Ala Gly Ala Gly Ala Gly Ser Gly Ala Gly Ala Gly Ser Gly
            260                 265                 270

Ala Gly Ala Gly Ala Gly Ser Gly Ala Gly Ala Gly Gly Ala Gly Ala
        275                 280                 285

Ala Ala Gly Ala Gly Ala Gly Ala Gly Ser Gly Ala Gly Ser Gly Ser
    290                 295                 300

Gly Ala Gly Ala Gly Ala Gly Ser Gly Ala Val Ala Gly Ser Gly Ala
305                 310                 315                 320

Gly Ser Gly Ala Ala Gly Gly Ala Gly Ala Gly Ala Gly Ala Gly Ala
                325                 330                 335

Ala Ala Ala Ala Ala Ala Ala Ala Glu Ala Ala Ala Ala Ala Ala Ala
            340                 345                 350

Ala Ala Ala Ala Ala Gly Ser Gly Ala Gly Ala Gly Gly Ala Gly Gly
        355                 360                 365

Tyr Gly Ala Gly Ala Gly Ala Gly Ala Gly Ala Gly Val Gly Gly Ala
    370                 375                 380

Gly Gly Ala Gly Gly Ala Gly Gly Ala Gly Gly Ala Gly Gly Tyr Gly
385                 390                 395                 400

Gly Ala Gly Val Val Tyr Val Ser Ala Gly Gly Ala Gly Ala Gly Ala
                405                 410                 415

Gly Ser Gly Ala Gly Ala Gly Ser Gly Ala Gly Ala Gly Ala Gly Ser
            420                 425                 430

Gly Ala Gly Ala Gly Gly Ala Gly Ala Ala Ala Gly Ala Gly Ala Gly
        435                 440                 445

Ala Gly Ser Gly Ala Gly Ser Gly Ser Gly Ala Gly Ala Gly Ala Gly
    450                 455                 460

Ser Gly Ala Val Ala Gly Ser Gly Ala Gly Ser Gly Ala Ala Gly Gly
465                 470                 475                 480

Ala Gly Ala Gly Ala Gly Ala Gly Ala Ala Ala Ala Ala Ala Ala Ala
                485                 490                 495

Ala Glu Ala Ala Ala Ala Ala Ala Ala Ala Ala Ala Ala Ala Gly Ser
            500                 505                 510

Gly Ala Gly Ala Gly Gly Ala Gly Gly Tyr Gly Ala Gly Ala Gly Ala
        515                 520                 525

Gly Ala Gly Ala Gly Ala Gly Gly Ala Ser Gly Gly Tyr Gly Ser Tyr
    530                 535                 540

Gly Ser Gly Val Ala Ala Gly Ala Gly Ala Gly Ala Gly Val Gly Gly
545                 550                 555                 560

Ser Arg Gly Ala Gly Val Gly Ala Gly Val Gly Ala Gly Tyr Gly Ser
                565                 570                 575

Ala Leu Asn Ser Gly Ala Gly Ala Gly Ala Gly Ala Gly Ala Gly Ala

580                 585                 590

Gly Gly Ala Ala Gly Ala Gly Ala Gly Ala Gly Ala Gly Ala Gly Ala
        595                 600                 605

Gly Ala Gly Phe Ala Ser Tyr Gly Arg Pro Gly Val Arg Gly Cys Gln
    610                 615                 620

Leu Ser Arg Lys Tyr Leu Leu Val Lys Val Gly Leu Arg Ser Thr Cys
625                 630                 635                 640

Ser Asp Cys

<210>  26
<211>  2163
<212>  DNA
<213>  Eumeta japonica

<400>  26
atggcgaagg cggattggta tgaagattgg aaaaaaaacc aaggttcatt tagagaaaca      60

gacttagcag acactgacga atatcaaaca gatagtaatg gtacaatgtt tgaaaaaaaa     120

acaacaagaa aaaagttcga aaaagatgga agtactatgg taaacagtga ttccggagaa     180

gataaaattg tacgaacttt cgtcgtggaa actgacgcat caggacatga agttatttat     240

gaagaagatg tagtcattaa aaaagttcca ggtaaacgga agaaagtttc acaggcaaat     300

gctaaagcta gtgctatagc agccagtgta ggtgcgggcg ttggagcagc ttccgttgct     360

ggtgcaggaa caggagctgg ctcaggtgca gctggtggag caggagcagg tgctgacgct     420

ggcgctgcag ccgccgctgc ggcagcagca caagcagcag ccgcagctgc tgccgccgca     480

ggtagtggcg ctggtgctgg acgagtcgga gcttacggac cctacggagg tttagcaagc     540

gctggtgctg gtggtgctgg cggagccggt ggtgcaggtg gatacggtgg tgcaagtgtc     600

gtctacgtag gtggcggagg agccggagca ggtgccggtt caggcgccgg tgctggatcg     660

ggtgccggag caggagctgg atcaggtgca ggagcaggtg gagcaggagc cgcagccgga     720

gctggtgctg gtgctggttc aggtgcagga tccggttcag gtgcgggcgc tggagcaggt     780

tccggagcag gagcaggttc cggtgctggt gcaggagcag gagctggctc aggtgcagca     840

ggtggagcag gagcaggagc tggcgctggc gctggcgctg cagccgcagc cgcagcagca     900

gcagaagcgg cagccgcagc tgccgccgcc gctgccgccg caggtagtgg agcaggcgct     960

ggaggagccg gaggctacgg agcgggagct ggagcaggtg ccggtgcagg tgctggcggc    1020

gctgctggtg ctggtggtgc tggcggagcc ggtggtgcag gtggatacgg tggtgctagc    1080

gtcgtctacg taagtgctgg aggagccgga gcaggtgccg gttcaggcgc cggtgctgga    1140

tcgggtgccg gagcaggagc tggatcaggt gcaggagcag gtggagcagg agccgcagcc    1200

ggagctggtg ctggtgctgg ttcaggtgca ggatccggtt caggtgcggg cgctggagca 1260

ggttccggag cagtagcagg ttccggtgct ggttcaggtg cagcaggtgg agcaggagct 1320

ggcgctggcg ctggcgctgc agccgcagcc gcagcagcag cagaagcggc agccgcagct 1380

gccgccgccg ctgccgccgc aggtagtgga gcaggcgctg gaggagccgg aggctacgga 1440

gcgggagctg gagcaggtgc cggagcaggt gttggcggcg ctggtggtgc tggtggtgct 1500

ggcggagccg gtggtgcagg tggatacggt ggtgcaggcg tcgtctacgt aagtgctgga 1560

ggagccggag caggtgccgg ttcaggcgcc ggtgctggat cgggtgccgg agcaggagct 1620

ggatcaggtg caggagcagg tggagcagga gccgcagccg gagctggtgc tggtgctggt 1680

tcaggtgcag gatccggttc aggtgcgggc gctggagcag gttccggagc agtagcaggt 1740

tccggtgctg gttcaggtgc agcaggtgga gcaggagctg gcgctggcgc tggcgctgca 1800

gccgcagccg cagcagcagc agaagcggca gccgcagctg ccgccgccgc tgccgccgca 1860

ggtagtggag caggcgctgg aggagccgga ggctacggag cgggagctgg agcaggtgcc 1920

ggtgcaggtg ctggcggcgc atctggaggc tacggttctt acggatcggg agttgcagca 1980

ggtgccggtg caggagctgg tgttggtggt agcaggggag caggtgttgg cgctggtgtt 2040

ggtgctggtt atggctccgc attgaattca ggagccggtg ccggagcagg tgctggtgct 2100

ggagctggtg gtgctgcagg agctggtgct ggtgcaggag caggagctgg cgctggagct 2160

gga 2163

<210> 27
<211> 721
<212> PRT
<213> Eumeta japonica

<400> 27

Met Ala Lys Ala Asp Trp Tyr Glu Asp Trp Lys Lys Asn Gln Gly Ser
1               5                   10                  15

Phe Arg Glu Thr Asp Leu Ala Asp Thr Asp Glu Tyr Gln Thr Asp Ser
            20                  25                  30

Asn Gly Thr Met Phe Glu Lys Lys Thr Thr Arg Lys Lys Phe Glu Lys
        35                  40                  45

Asp Gly Ser Thr Met Val Asn Ser Asp Ser Gly Glu Asp Lys Ile Val
    50                  55                  60

Arg Thr Phe Val Val Glu Thr Asp Ala Ser Gly His Glu Val Ile Tyr
65                  70                  75                  80

Glu Glu Asp Val Val Ile Lys Lys Val Pro Gly Lys Arg Lys Lys Val
                    85                  90                  95

Ser Gln Ala Asn Ala Lys Ala Ser Ala Ile Ala Ala Ser Val Gly Ala
            100                 105                 110

Gly Val Gly Ala Ala Ser Val Ala Gly Ala Gly Thr Gly Ala Gly Ser
        115                 120                 125

Gly Ala Ala Gly Gly Ala Gly Ala Gly Ala Asp Ala Gly Ala Ala Ala
    130                 135                 140

Ala Ala Ala Ala Ala Ala Gln Ala Ala Ala Ala Ala Ala Ala Ala Ala
145                 150                 155                 160

Gly Ser Gly Ala Gly Ala Gly Arg Val Gly Ala Tyr Gly Pro Tyr Gly
                165                 170                 175

Gly Leu Ala Ser Ala Gly Ala Gly Gly Ala Gly Gly Ala Gly Gly Ala
            180                 185                 190

Gly Gly Tyr Gly Gly Ala Ser Val Val Tyr Val Gly Gly Gly Gly Ala
        195                 200                 205

Gly Ala Gly Ala Gly Ser Gly Ala Gly Ala Gly Ser Gly Ala Gly Ala
    210                 215                 220

Gly Ala Gly Ser Gly Ala Gly Ala Gly Gly Ala Gly Ala Ala Ala Gly
225                 230                 235                 240

Ala Gly Ala Gly Ala Gly Ser Gly Ala Gly Ser Gly Ser Gly Ala Gly
                245                 250                 255

Ala Gly Ala Gly Ser Gly Ala Gly Ala Gly Ser Gly Ala Gly Ala Gly
            260                 265                 270

Ala Gly Ala Gly Ser Gly Ala Ala Gly Gly Ala Gly Ala Gly Ala Gly
        275                 280                 285

Ala Gly Ala Gly Ala Ala Ala Ala Ala Ala Ala Ala Ala Glu Ala Ala
    290                 295                 300

Ala Ala Ala Ala Ala Ala Ala Ala Ala Ala Gly Ser Gly Ala Gly Ala
305                 310                 315                 320

Gly Gly Ala Gly Gly Tyr Gly Ala Gly Ala Gly Ala Gly Ala Gly Ala
                325                 330                 335

Gly Ala Gly Gly Ala Ala Gly Ala Gly Gly Ala Gly Gly Ala Gly Gly
            340                 345                 350

Ala Gly Gly Tyr Gly Gly Ala Ser Val Val Tyr Val Ser Ala Gly Gly
        355                 360                 365

Ala Gly Ala Gly Ala Gly Ser Gly Ala Gly Ala Gly Ser Gly Ala Gly
    370                 375                 380

Ala Gly Ala Gly Ser Gly Ala Gly Ala Gly Gly Ala Gly Ala Ala Ala
385                 390                 395                 400

Gly Ala Gly Ala Gly Ala Gly Ser Gly Ala Gly Ser Gly Ser Gly Ala
                405                 410                 415

Gly Ala Gly Ala Gly Ser Gly Ala Val Ala Gly Ser Gly Ala Gly Ser
            420                 425                 430

Gly Ala Ala Gly Gly Ala Gly Ala Gly Ala Gly Ala Gly Ala Ala Ala
        435                 440                 445

Ala Ala Ala Ala Ala Ala Glu Ala Ala Ala Ala Ala Ala Ala Ala Ala
    450                 455                 460

Ala Ala Ala Gly Ser Gly Ala Gly Ala Gly Gly Ala Gly Gly Tyr Gly
465                 470                 475                 480

Ala Gly Ala Gly Ala Gly Ala Gly Ala Gly Val Gly Gly Ala Gly Gly
                485                 490                 495

Ala Gly Gly Ala Gly Gly Ala Gly Gly Ala Gly Gly Tyr Gly Gly Ala
            500                 505                 510

Gly Val Val Tyr Val Ser Ala Gly Gly Ala Gly Ala Gly Ala Gly Ser
        515                 520                 525

Gly Ala Gly Ala Gly Ser Gly Ala Gly Ala Gly Ala Gly Ser Gly Ala
    530                 535                 540

Gly Ala Gly Gly Ala Gly Ala Ala Ala Gly Ala Gly Ala Gly Ala Gly
545                 550                 555                 560

Ser Gly Ala Gly Ser Gly Ser Gly Ala Gly Ala Gly Ala Gly Ser Gly
                565                 570                 575

Ala Val Ala Gly Ser Gly Ala Gly Ser Gly Ala Ala Gly Gly Ala Gly
            580                 585                 590

Ala Gly Ala Gly Ala Gly Ala Ala Ala Ala Ala Ala Ala Ala Ala Glu
        595                 600                 605

Ala Ala Ala Ala Ala Ala Ala Ala Ala Ala Ala Ala Gly Ser Gly Ala
    610                 615                 620

Gly Ala Gly Gly Ala Gly Gly Tyr Gly Ala Gly Ala Gly Ala Gly Ala
625                 630                 635                 640

Gly Ala Gly Ala Gly Gly Ala Ser Gly Gly Tyr Gly Ser Tyr Gly Ser
                645                 650                 655

Gly Val Ala Ala Gly Ala Gly Ala Gly Ala Gly Val Gly Gly Ser Arg
            660                 665                 670

Gly Ala Gly Val Gly Ala Gly Val Gly Ala Gly Tyr Gly Ser Ala Leu
        675                 680                 685

Asn Ser Gly Ala Gly Ala Gly Ala Gly Ala Gly Ala Gly Ala Gly Gly
    690                 695                 700

Ala Ala Gly Ala Gly Ala Gly Ala Gly Ala Gly Ala Gly Ala Gly Ala
705                 710                 715                 720

Gly

<210>  28
<211>  1833
<212>  DNA
<213>  Eumeta japonica

<400>  28
atggcgggcg ttggagcagc ttccgttgct ggtgcaggaa caggagctgg ctcaggtgca      60

gctggtggag caggagcagg tgctgacgct ggcgctgcag ccgccgctgc ggcagcagca     120

caagcagcag ccgcagctgc tgccgccgca ggtagtggcg ctggtgctgg acgagtcgga     180

gcttacggac cctacggagg tttagcaagc gctggtgctg gtggtgctgg cggagccggt     240

ggtgcaggtg gatacggtgg tgcaagtgtc gtctacgtag gtggcggagg agccggagca     300

ggtgccggtt caggcgccgg tgctggatcg ggtgccggag caggagctgg atcaggtgca     360

ggagcaggtg gagcaggagc cgcagccgga gctggtgctg gtgctggttc aggtgcagga     420

tccggttcag gtgcgggcgc tggagcaggt tccggagcag gagcaggttc cggtgctggt     480

gcaggagcag gagctggctc aggtgcagca ggtggagcag gagcaggagc tggcgctggc     540

gctggcgctg cagccgcagc cgcagcagca gcagaagcgg cagccgcagc tgccgccgcc     600

gctgccgccg caggtagtgg agcaggcgct ggaggagccg gaggctacgg agcgggagct     660

ggagcaggtg ccggtgcagg tgctggcggc gctgctggtg ctggtggtgc tggcggagcc     720

ggtggtgcag gtggatacgg tggtgctagc gtcgtctacg taagtgctgg aggagccgga     780

gcaggtgccg gttcaggcgc cggtgctgga tcgggtgccg gagcaggagc tggatcaggt     840

gcaggagcag gtggagcagg agccgcagcc ggagctggtg ctggtgctgg ttcaggtgca     900

ggatccggtt caggtgcggg cgctggagca ggttccggag cagtagcagg ttccggtgct     960

ggttcaggtg cagcaggtgg agcaggagct ggcgctggcg ctggcgctgc agccgcagcc    1020

gcagcagcag cagaagcggc agccgcagct gccgccgccg ctgccgccgc aggtagtgga    1080

gcaggcgctg gaggagccgg aggctacgga gcgggagctg gagcaggtgc cggagcaggt    1140

gttggcggcg ctggtggtgc tggtggtgct ggcggagccg gtggtgcagg tggatacggt    1200

ggtgcaggcg tcgtctacgt aagtgctgga ggagccggag caggtgccgg ttcaggcgcc    1260

ggtgctggat cgggtgccgg agcaggagct ggatcaggtg caggagcagg tggagcagga    1320

gccgcagccg gagctggtgc tggtgctggt tcaggtgcag gatccggttc aggtgcgggc    1380

gctggagcag gttccggagc agtagcaggt tccggtgctg gttcaggtgc agcaggtgga    1440

gcaggagctg gcgctggcgc tggcgctgca gccgcagccg cagcagcagc agaagcggca    1500

gccgcagctg ccgccgccgc tgccgccgca ggtagtggag caggcgctgg aggagccgga    1560

ggctacggag cgggagctgg agcaggtgcc ggtgcaggtg ctggcggcgc atctggaggc    1620

tacggttctt acggatcggg agttgcagca ggtgccggtg caggagctgg tgttggtggt    1680

agcaggggag caggtgttgg cgctggtgtt ggtgctggtt atggctccgc attgaattca    1740

ggagccggtg ccggagcagg tgctggtgct ggagctggtg gtgctgcagg agctggtgct    1800

ggtgcaggag caggagctgg cgctggagct gga                                 1833

<210>  29
<211>  611
<212>  PRT
<213>  Eumeta japonica

<400>  29

Met Ala Gly Val Gly Ala Ala Ser Val Ala Gly Ala Gly Thr Gly Ala
1               5                   10                  15

Gly Ser Gly Ala Ala Gly Gly Ala Gly Ala Gly Ala Asp Ala Gly Ala
            20                  25                  30

Ala Ala Ala Ala Ala Ala Ala Ala Gln Ala Ala Ala Ala Ala Ala Ala
        35                  40                  45

Ala Ala Gly Ser Gly Ala Gly Ala Gly Arg Val Gly Ala Tyr Gly Pro
    50                  55                  60

Tyr Gly Gly Leu Ala Ser Ala Gly Ala Gly Gly Ala Gly Gly Ala Gly
65                  70                  75                  80

Gly Ala Gly Gly Tyr Gly Gly Ala Ser Val Val Tyr Val Gly Gly Gly
                85                  90                  95

Gly Ala Gly Ala Gly Ala Gly Ser Gly Ala Gly Ala Gly Ser Gly Ala
            100                 105                 110

Gly Ala Gly Ala Gly Ser Gly Ala Gly Ala Gly Gly Ala Gly Ala Ala
        115                 120                 125

Ala Gly Ala Gly Ala Gly Ala Gly Ser Gly Ala Gly Ser Gly Ser Gly
    130                 135                 140

Ala Gly Ala Gly Ala Gly Ser Gly Ala Gly Ala Gly Ser Gly Ala Gly
145                 150                 155                 160

Ala Gly Ala Gly Ala Gly Ser Gly Ala Ala Gly Gly Ala Gly Ala Gly
                165                 170                 175

Ala Gly Ala Gly Ala Gly Ala Ala Ala Ala Ala Ala Ala Ala Ala Glu
            180                 185                 190

Ala Ala Ala Ala Ala Ala Ala Ala Ala Ala Ala Ala Gly Ser Gly Ala
        195                 200                 205

Gly Ala Gly Gly Ala Gly Gly Tyr Gly Ala Gly Ala Gly Ala Gly Ala
    210                 215                 220

Gly Ala Gly Ala Gly Gly Ala Ala Gly Ala Gly Gly Ala Gly Gly Ala
225                 230                 235                 240

Gly Gly Ala Gly Gly Tyr Gly Gly Ala Ser Val Val Tyr Val Ser Ala
                245                 250                 255

Gly Gly Ala Gly Ala Gly Ala Gly Ser Gly Ala Gly Ala Gly Ser Gly
            260                 265                 270

Ala Gly Ala Gly Ala Gly Ser Gly Ala Gly Ala Gly Gly Ala Gly Ala
        275                 280                 285

Ala Ala Gly Ala Gly Ala Gly Ala Gly Ser Gly Ala Gly Ser Gly Ser

290                 295                 300

Gly Ala Gly Ala Gly Ala Gly Ser Gly Ala Val Ala Gly Ser Gly Ala
305                 310                 315                 320

Gly Ser Gly Ala Ala Gly Gly Ala Gly Ala Gly Ala Gly Ala Gly Ala
                325                 330                 335

Ala Ala Ala Ala Ala Ala Ala Ala Glu Ala Ala Ala Ala Ala Ala Ala
            340                 345                 350

Ala Ala Ala Ala Ala Gly Ser Gly Ala Gly Ala Gly Gly Ala Gly Gly
        355                 360                 365

Tyr Gly Ala Gly Ala Gly Ala Gly Ala Gly Ala Gly Val Gly Gly Ala
    370                 375                 380

Gly Gly Ala Gly Gly Ala Gly Gly Ala Gly Gly Ala Gly Gly Tyr Gly
385                 390                 395                 400

Gly Ala Gly Val Val Tyr Val Ser Ala Gly Gly Ala Gly Ala Gly Ala
                405                 410                 415

Gly Ser Gly Ala Gly Ala Gly Ser Gly Ala Gly Ala Gly Ala Gly Ser
            420                 425                 430

Gly Ala Gly Ala Gly Gly Ala Gly Ala Ala Ala Gly Ala Gly Ala Gly
        435                 440                 445

Ala Gly Ser Gly Ala Gly Ser Gly Ser Gly Ala Gly Ala Gly Ala Gly
    450                 455                 460

Ser Gly Ala Val Ala Gly Ser Gly Ala Gly Ser Gly Ala Ala Gly Gly
465                 470                 475                 480

Ala Gly Ala Gly Ala Gly Ala Gly Ala Ala Ala Ala Ala Ala Ala Ala
                485                 490                 495

Ala Glu Ala Ala Ala Ala Ala Ala Ala Ala Ala Ala Ala Ala Gly Ser
            500                 505                 510

Gly Ala Gly Ala Gly Gly Ala Gly Gly Tyr Gly Ala Gly Ala Gly Ala
        515                 520                 525

Gly Ala Gly Ala Gly Ala Gly Gly Ala Ser Gly Gly Tyr Gly Ser Tyr
    530                 535                 540

Gly Ser Gly Val Ala Ala Gly Ala Gly Ala Gly Ala Gly Val Gly Gly
545                 550                 555                 560

Ser Arg Gly Ala Gly Val Gly Ala Gly Val Gly Ala Gly Tyr Gly Ser
                565                 570                 575

Ala Leu Asn Ser Gly Ala Gly Ala Gly Ala Gly Ala Gly Ala Gly Ala
            580                 585                 590

Gly Gly Ala Ala Gly Ala Gly Ala Gly Ala Gly Ala Gly Ala Gly Ala
        595                 600                 605

Gly Ala Gly
    610

<210>  30
<211>  22
<212>  DNA
<213>  Artificial

<220>
<223>  primer

<400>  30
atgagagctc tgaccttcgt ga                                                22

<210>  31
<211>  21
<212>  DNA
<213>  Artificial

<220>
<223>  primer

<400>  31
accaccgtat ccacctgcac c                                                 21

<210>  32
<211>  1608
<212>  DNA
<213>  Eumeta japonica

<400>  32
atgagagctc tgaccttcgt gatcctgtgc tgcgctttgc agcaatatgc aacagcaaag      60

gcggattggt atgaagattg gaaaaaaaac caaggttcat ttagagaaac agacttagca      120

gacactgacg aatatcaaac agatagtaat ggtacaatgt ttgaaaaaaa aacaacaaga      180

aaaaagttcg aaaaagatgg aagtactatg gtaaacagtg attccggaga agataaaatt      240

gtacgaactt tcgtcgtgga aactgacgca tcaggacatg aagttattta tgaagaagat      300

gtagtcatta aaaaagttcc aggtaaacgg aagaaagttt cacaggcaaa tgctaaagct      360

agtgctatag cagccagtgt aggtgcgggc gttggagcag cttccgttgc tggtgcagga      420

acaggagctg gctcaggtgc agctggtgga gcaggagcag gtgctgacgc tggcgctgca 480

gccgccgctg cggcagcagc acaagcagca gccgcagctg ctgccgccgc aggtagtggc 540

gctggtgctg gacgagtcgg agcttacgga ccctacggag gtttagcaag cgctggtgct 600

ggtggtgctg gcggagccgg tggtgcaggt ggatacggtg gtgcaagtgt cgtctacgta 660

ggtggcggag gagccggagc aggtgccggt tcaggcgccg gtgctggatc gggtgccgga 720

gcaggagctg gatcaggtgc aggagcaggt ggagcaggag ccgcagccgg agctggtgct 780

ggtgctggtt caggtgcagg atccggttca ggtgcgggcg ctggagcagg ttccggagca 840

ggagcaggtt ccggtgctgg tgcaggagca ggagctggct caggtgcagc aggtggagca 900

ggagcaggag ctggcgctgg cgctggcgct gcagccgcag ccgcagcagc agcagaagcg 960

gcagccgcag ctgccgccgc cgctgccgcc gcaggtagtg gagcaggcgc tggaggagcc 1020

ggaggctacg gagcgggagc tggagcaggt gccggtgcag gtgctggcgg cgctgctggt 1080

gctggtggtg ctggcggagc cggtggtgca ggtggatacg gtggtgcaag tgtcgtctac 1140

gtaggtggcg gaggagccgg agcaggtgcc ggttcaggcg ccggtgctgg atcgggtgcc 1200

ggagcaggag ctggatcagg tgctggagca ggtagagcag gagccgcagc cggagctggt 1260

gctggtgctg gttcaggtgc aggatccggt tcaggtgcgg gcgctggagc aggttccgga 1320

gcaggagcag gttccggtgc tggtgcagga gcaggagctg gttcaggtgc agcaggtgga 1380

gcaggagcag gagctggcgc tggcgctggc gctgcagccg cagccgcagc agcagcagaa 1440

gcggcagccg cagctgccgc cgccgctgcc gccgcaggta gtggagcagg cgctggagga 1500

gccggaggct acggagcggg agctggagca ggtgccggtg caggtgctgg cggcgctgct 1560

ggtgctggtg gtgctggcgg agccggtggt gcaggtggat acggtggt 1608

<210> 33
<211> 40
<212> DNA
<213> Artificial

<220>
<223> primer

<400> 33
atagaattcc catggcgaag gcggattggt atgaagattg 40

<210> 34
<211> 38
<212> DNA
<213> Artificial

<220>
<223> primer

<400> 34

ataggatccg ctagcaccac cgtatccacc tgcaccac 38

<210> 35
<211> 585
<212> DNA
<213> Eumeta japonica

<400> 35
aaggcggatt ggtatgaaga ttggaaaaaa aaccaaggtt catttagaga aacagactta 60

gcagacactg acgaatatca aacagatagt aatggtacaa tgtttgaaaa aaaaacaaca 120

agaaaaaagt tcgaaaaaga tggaagtact atggtaaaca gtgattccgg agaagataaa 180

attgtacgaa ctttcgtcgt ggaaactgac gcatcaggac atgaagttat ttatgaagaa 240

gatgtagtca ttaaaaaagt tccaggtaaa cggaagaaag tttcacaggc aaatgctaaa 300

gctagtgcta tagcagccag tgtaggtgcg ggcgttggag cagcttccgt tgctggtgca 360

ggaacaggag ctggctcagg tgcagctggt ggagcaggag caggtgctga cgctggcgct 420

gcagccgccg ctgcggcagc agcacaagca gcagccgcag ctgctgccgc cgcaggtagt 480

ggcgctggtg ctggacgagt cggagcttac ggaccctacg gaggtttagc aagcgctggt 540

gctggtggtg ctggcggagc cggtggtgca ggtggatacg gtggt 585

<210> 36
<211> 1068
<212> DNA
<213> Eumeta japonica

<400> 36
aaggcggatt ggtatgaaga ttggaaaaaa aaccaaggtt catttagaga aacagactta 60

gcagacactg acgaatatca aacagatagt aatggtacaa tgtttgaaaa aaaaacaaca 120

agaaaaaagt tcgaaaaaga tggaagtact atggtaaaca gtgattccgg agaagataaa 180

attgtacgaa ctttcgtcgt ggaaactgac gcatcaggac atgaagttat ttatgaagaa 240

gatgtagtca ttaaaaaagt tccaggtaaa cggaagaaag tttcacaggc aaatgctaaa 300

gctagtgcta tagcagccag tgtaggtgcg ggcgttggag cagcttccgt tgctggtgca 360

ggaacaggag ctggctcagg tgcagctggt ggagcaggag caggtgctga cgctggcgct 420

gcagccgccg ctgcggcagc agcacaagca gcagccgcag ctgctgccgc cgcaggtagt 480

ggcgctggtg ctggacgagt cggagcttac ggaccctacg gaggtttagc aagcgctggt 540

gctggtggtg ctggcggagc cggtggtgca ggtggatacg gtggtgcaag tgtcgtctac 600

gtaggtggcg gaggagccgg agcaggtgcc ggttcaggcg ccggtgctgg atcgggtgcc 660

ggagcaggag ctggatcagg tgcaggagca ggtggagcag gagccgcagc cggagctggt 720

gctggtgctg gttcaggtgc aggatccggt tcaggtgcgg gcgctggagc aggttccgga 780

gcaggagcag gttccggtgc tggtgcagga gcaggagctg gctcaggtgc agcaggtgga 840

gcaggagcag gagctggcgc tggcgctggc gctgcagccg cagccgcagc agcagcagaa 900

gcggcagccg cagctgccgc cgccgctgcc gccgcaggta gtggagcagg cgctggagga 960

gccggaggct acggagcggg agctggagca ggtgccggtg caggtgctgg cggcgctgct 1020

ggtgctggtg gtgctggcgg agccggtggt gcaggtggat acggtggt 1068

<210> 37
<211> 31
<212> DNA
<213> Artificial

<220>
<223> primer

<400> 37
atagctagcg tcgtctacgt aagtgctgga g 31

<210> 38
<211> 42
<212> DNA
<213> Artificial

<220>
<223> primer

<400> 38
tataagcttc tcgagacaat ctgagcatgt ggatcttaaa cc 42

<210> 39
<211> 1179
<212> DNA
<213> Eumeta japonica

<400> 39
gtcgtctacg taagtgctgg aggagccgga gcaggtgccg gttcaggcgc cggtgctgga 60

tcgggtgccg gagcaggagc tggatcaggt gcaggagcag gtggagcagg agccgcagcc 120

ggagctggtg ctggtgctgg ttcaggtgca ggatccggtt caggtgcggg cgctggagca 180

ggttccggag cagtagcagg ttccggtgct ggttcaggtg cagcaggtgg agcaggagct 240

ggcgctggcg ctggcgctgc agccgcagcc gcagcagcag cagaagcggc agccgcagct 300

gccgccgccg ctgccgccgc aggtagtgga gcaggcgctg gaggagccgg aggctacgga 360

gcgggagctg gagcaggtgc cggagcaggt gttggcggcg ctggtggtgc tggtggtgct 420

ggcggagccg gtggtgcagg tggatacggt ggtgcaggcg tcgtctacgt aagtgctgga 480

ggagccggag caggtgccgg ttcaggcgcc ggtgctggat cgggtgccgg agcaggagct 540

ggatcaggtg caggagcagg tggagcagga gccgcagccg gagctggtgc tggtgctggt 600

tcaggtgcag gatccggttc aggtgcgggc gctggagcag gttccggagc agtagcaggt 660

tccggtgctg gttcaggtgc agcaggtgga gcaggagctg gcgctggcgc tggcgctgca 720

gccgcagccg cagcagcagc agaagcggca gccgcagctg ccgccgccgc tgccgccgca 780

ggtagtggag caggcgctgg aggagccgga ggctacggag cgggagctgg agcaggtgcc 840

ggtgcaggtg ctggcggcgc atctggaggc tacggttctt acggatcggg agttgcagca 900

ggtgccggtg caggagctgg tgttggtggt agcaggggag caggtgttgg cgctggtgtt 960

ggtgctggtt atggctccgc attgaattca ggagccggtg ccggagcagg tgctggtgct 1020

ggagctggtg gtgctgcagg agctggtgct ggtgcaggag caggagctgg cgctggagct 1080

ggattcgctt cttatggaag accaggtgtt cgtggatgtc aactgtctcg taaatacctt 1140

ttggttaaag ttggtttaag atccacatgc tcagattgt 1179

<210> 40
<211> 34
<212> DNA
<213> Artificial

<220>
<223> primer

<400> 40
atagaattcc catggcgggc gttggagcag cttc 34

<210> 41
<211> 37
<212> DNA
<213> Artificial

<220>
<223> primer

<400> 41
tataagcttc tcgagtccag ctccagcgcc agctcct 37

<210> 42
<211> 34
<212> DNA
<213> Artificial

<220>
<223> primer

<400> 42
atagaattcc catggcgggc gttggaacag cttc 34

<210> 43
<211> 42
<212> DNA
<213> Artificial

<220>
<223> primer

<400> 43

tataagcttc tcgagacaat ctgagcatgt ggatcttaaa cc                42

<210>  44
<211>  5
<212>  PRT
<213>  Eumeta japonica

<400>  44

Ser Val Val Tyr Val
1               5

<210>  45
<211>  5
<212>  PRT
<213>  Eumeta japonica

<400>  45

Val Val Tyr Val Ser
1               5

<210>  46
<211>  7
<212>  PRT
<213>  Eumeta japonica

<400>  46

Ser Thr Leu Ile Ile Val Asp
1               5

<210>  47
<211>  22
<212>  PRT
<213>  Escherichia coli

<400>  47

Met Lys Tyr Leu Leu Pro Thr Ala Ala Ala Gly Leu Leu Leu Leu Ala
1               5                   10                  15

Ala Gln Pro Ala Met Ala
            20

<210>  48
<211>  66
<212>  DNA
<213>  Escherichia coli

<400>  48
atgaaatacc tgctgccgac cgctgctgct ggtctgctgc tcctcgctgc ccagccggcg        60

atggcc                                                                    66

## Description

### Technical Field

The present invention relates to a gene expression enhancement system for increasing the production output of a fibroin H chain protein that is a main fibrous component of a bagworm silk produced by using gene recombination technology, and to a method of producing a recombinant bagworm fibroin H chain protein using the system.

### Background Art

Threads constituting insect cocoons and hairs of mammals have been used as animal fibers for clothes and the like since ancient times. Especially silks from silk moth (*Bombyx mori*) larvae, namely a silkworm, (herein often referred to as "silkworm silk") has excellent properties for absorption and desorption of moisture, moisture retention, and heat retention, and also has a unique gloss and smooth texture, and these features make silk a valuable and expensive natural material even today.

However, there exist animal fibers in nature having properties comparable or superior to those of silkworm silks. For example, one of such fibers is the thread spun by a bagworm (the thread is herein often referred to as "bagworm silk"). The bagworm is a general term referring to larvae of moths belonging to the family *Psychidae* in the order *Lepidoptera* and are known to spend the entire larval stage living with spindle-shaped or cylinder-shaped nests (bag nests) made of pieces of leaves and twigs and assembled with threads. For example, the larvae usually hide themselves inside the nests and move with the nests even during feeding.

The silk of bagworm has mechanical properties superior to those of silkworm silk. For example, bagworm silks from *Eumeta minuscula* have an elastic modulus up to 3.5 times of that of silkworm silks, and have a very high strength (Non-Patent Literatures 1 and 2). Additionally, a single fiber of bagworm silk has a cross-sectional area only about one-seventh of that of a single fiber of silkworm silk, which allows production of fine, thin and light fabrics with a smooth texture. Moreover, the bagworm silk has a gloss and a shiny appearance comparable or superior to those of a silkworm silk. Thus, the bagworm silk can be an animal fiber which is highly promising as a novel natural material.

However, there are several problems to be solved in practical use of the bagworm silk. One of the problems is a problem with mass production. For practical use of the bagworm silk as a fiber material, it is indispensable to obtain a large number of bagworm nests. However, the number of bagworm nests that can be collected in the field is not sufficient for mass production. Thus, it is impossible to avoid methods for breeding bagworms on a large scale and for efficiently collecting bagworm silks.

For practical use of the bagworm silk, another important problem is the purity of the bagworm silk. Contaminants, such as pieces of leaves and twigs are inevitably attached on the surface of bagworm nests. In terms of quality, these contaminants have to be completely removed for practical use of bagworm silk as a fiber material. However, the removing work requires enormous labor and cost, and complete removal of the contaminants from bagworm nests is difficult with existing techniques. Thus, so far, it was only possible to obtain bagworm silks that cost much, and furthermore, were stained with pigments from the contaminants, and thus having low quality.

As a solution for the above-mentioned problems, the methods of efficiently collecting high purity bagworm silks from bagworms have been currently developed, as disclosed in Patent Literature 1 and Patent Literature 2. However, the development of bagworm silk industry has just begun. Facilities and systems for mass production of bagworm silks are under development, and still a great amount of time will be taken to achieve mass production of bagworm silks.

As a solution for the above-mentioned problems, there is a method of mass-producing a recombinant bagworm silk using gene recombination technology, besides a method of collecting a silk directly from a bagworm. Introduction of a cloned bagworm silk gene into a host to allow the gene to be expressed makes it possible to mass-produce recombinant bagworm silk in the host cell. However, this production method has an essential problem in that the whole genome sequence of the bagworm silk has not yet been determined although critically important. This is because the fibroin H chain protein (Fib H protein), which is the main fibrous component of the silk, has an amino acid sequence composed of many repeated glycine residues and alanine residues, thus making it extremely difficult to determine the full-length base sequence of the fibroin H chain gene (Fib H gene) using conventional cloning techniques. In order to solve this problem, in Patent Literature 3, a recombinant chimera Fib H gene consisting of a bagworm Fib H (herein often referred to as "bFib H") gene and a silkworm Fib H (herein often referred to as "sFib H") gene has been produced. This chimera Fib H gene is an artificial gene having the full length obtained by joining a part of the bFib H gene information identified in *Eumeta japonica* by transcriptome analysis and a part of the existing sFib H gene information. Patent Literature 3 discloses a method of producing a chimera silk having the physical properties of a bagworm silk in addition to those of a silkworm silk by introducing the chimera Fib H gene into a silkworm to create a recombinant silkworm and make the recombinant silkworm spin a thread.

### Citation List

### Patent Literature

Patent Literature 1: JP2018-197415A
Patent Literature 2: JP2019-013207A
Patent Literature 3: JP2018-074403A

### Non-Patent Literature

Non-Patent Literature 1: Shigeyosi Ohsaki, 2002, Sen'i Gakkaishi (Sen'i To Kogyo), 58: 74-78.
Non-Patent Literature 2: Gosline J. M., *et al.,* 1999, 202, 3295-3303.

### Summary of Invention

### Technical Problem

As a host for mass-production-system of protein, microorganisms such as *E. coli* (*Escherichia coli*) and a yeast are generally used, besides the silkworms used in the above-mentioned Patent Literature 3, i.e., JP2018-074403. In view of this, the present inventors newly cloned the bagworm Fib H gene of *Eumeta japonica* to construct a bagworm Fib H gene expression system using a microorganism as the host for mass-production-system, and succeeded in obtaining a bagworm Fib H cDNA comprising a region that was unidentified until then. When the recombinant bagworm Fib H (herein often referred to as "rbFib H") gene obtained was inserted into a gene expression vector for a microorganism, and the vector was introduced into *E. coli* to make them produce the rbFib H protein, it was found that such an operation significantly reduces the expression level of the rbFib H protein. Although the specific cause of this phenomenon is unclear, it is assumed that some control effect of inhibiting the expression have been worked. However, such a reduction in the expression level can be a critical problem in a mass-production-system of proteins.

Therefore, an object of the present invention is to develop an rbFib H gene that makes it possible to mass-produce the rbFib H protein even in a microorganism expression system and the like, and to develop an expression vector comprising the rbFib H gene.

### Solution to Problem

To solve the above-mentioned problems, the present inventors have vigorously made studies, and consequently made new findings when a gene encoding an rbFib H protein of a one-terminal deletion type in which either of the N-terminal region or the C-terminal region of the rbFib H protein has been deleted is expressed, the expression level of the gene is markedly increased. In contrast, an rbFib H protein of a both-terminal deletion type in which only the central region contributive to the physical properties of the Fib H protein is remained, has exhibited an increase in the expression level, but simultaneously an increase in the degradation amount of the rbFib H protein. The present invention is based on these findings, and will provide the following items.
(1) A modified-rbFib H (m-rbFib H) protein obtained by modifying an rbFib H protein comprising an N-terminal region, a central region, and a C-terminal region in this order from the N-terminal side, said m-rbFib H protein deleting all or part of the N-terminal region or all or part of the C-terminal region thereof, wherein:
   the N-terminal region consists of an amino acid sequence shown in SEQ ID NO: 1, an amino acid sequence derived from the amino acid sequence shown in SEQ ID NO: 1 having an addition, a deletion, or a substitution of one or a plurality of amino acid(s), or an amino acid sequence having an amino acid identity of 90% or more to the amino acid sequence shown in SEQ ID NO: 1;
   the central region has three or more linked central repeat units consisting of the same and/or different amino acid sequences; and
   the C-terminal region consists of an amino acid sequence shown in SEQ ID NO: 2, an amino acid sequence derived from the amino acid sequence shown in SEQ ID NO: 2 having an addition, a deletion, or a substitution of one or a plurality of amino acid(s), or an amino acid sequence having an amino acid identity of 90% or more to the amino acid sequence shown in SEQ ID NO: 2;wherein said central repeat unit comprises 30 units or more of 2-amino-acid units consisting of glycine and alanine, comprises an Ala cluster comprising 15 to 25 alanines at the N-terminal side, and consists of 120 to 170 amino acids in full length.
(2) The m-rbFib H protein according to (1), wherein the Ala cluster consists of the amino acid sequence shown in SEQ ID NO: 3 or 4.
(3) The m-rbFib H protein according to (2), wherein the central repeat unit is selected from the amino acid sequences shown in SEQ ID NOs: 5 to 13.
(4) An m-rbFib H gene encoding the m-rbFib H protein according to any one of (1) to (3).
(5) The m-rbFib H gene according to (4), consisting of any one of the base sequences shown in SEQ ID NO: 16, 18, 24, or 26.
(6) An expression vector of an m-rbFib H gene, comprising the m-rbFib H gene according to (4) or (5) in a state that allows for the expression in a host cell.
(7) A transformant consisting of a host comprising the expression vector of the m-rbFib H gene according to (6), or a progeny thereof.
(8) The transformant or the progeny thereof according to (7), wherein the host is a microorganism or an insect culture cell.
(9) A method of producing an m-rbFib H protein, comprising:
   a culture process of culturing the transformant or the progeny thereof according to (7) or (8) under predetermined conditions; and
   a preparation process of preparing the m-rbFib H protein from the culture solution and/or the transformant after the culture process.

The present specification incorporates the disclosure of Japanese Patent Application No. 2019-097154, to which the present application claims priority.

### Advantageous Effects of Invention

According to the expression vector of an m-rbFib H gene of the present invention, m-rbFib H proteins can be mass-produced by introducing the expression vector into a host to induce the expression.

### Brief Description of Drawings

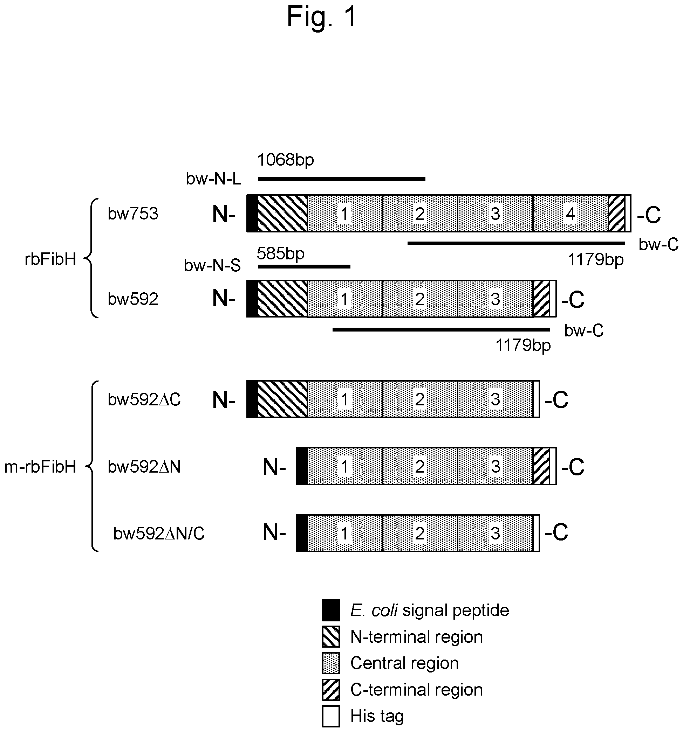

[Figure 1] Figure 1 shows the structural schematic diagrams of an rbFib H protein that is encoded by the rbFib H gene cloned in the present invention, and an m-rbFib H protein that is encoded by the m-rbFib H gene obtained by modifying the rbFib H gene. This Figure shows the bw753 protein and the bw592 protein constructed as rbFib H proteins in Examples, and in addition, the bw592ΔC protein, the bw592ΔN protein, and the bw592ΔN/C protein constructed as m-rbFib H proteins in Examples. The black bars in the Figure represent the amplification fragments of the rbFib H gene, which was used to clone the rbFib H gene, and represent the positions corresponding to the amino acid regions on m-rbFib H proteins, that are encoded by the fragments.

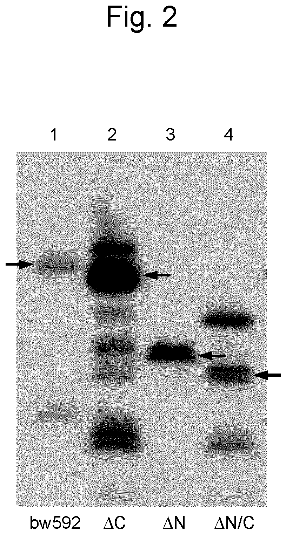

[Figure 2] Figure 2 is a Western blotting diagram of the rbFib H protein in each of the transformants in Example 4. Lane 1 represents the bw592 that is the rbFib H protein. Lane 2 represents the bw592ΔC that is the m-rbFib H protein. Lane 3 represents the bw592ΔN that is the m-rbFib H protein. Lane 4 represents the bw592ΔN/C that is the m-rbFib H protein. The arrows in the Figure represent the positions of the proteins of interest.

### Description of Embodiments

### 1. Modified-recombinant bagworm fibroin H chain protein

### 1-1. Overview

The first aspect of the present invention is a modified-recombinant bagworm fibroin H chain (herein often referred to as "m-rbFib H") protein. The m-rbFib H protein is a protein encoded by a modified-recombinant bagworm fibroin H chain gene (m-rbFib H gene) obtained by further modifying the rbFib H gene newly cloned by the present inventors.

According to the m-rbFib H gene of the present invention, it is possible to express recombinant proteins of 15 times or more compared to that of the rbFib H gene by expressing an expression vectors comprising the genes in host cells such as microorganism. This makes it possible to mass-produce rbFib H proteins using a microorganism gene expression system.

### 1-2. Definition of Terms

The following terms frequently used herein are defined as follows.

The term "bagworm" collectively refers to a moth larva belonging to the family *Psychidae* in the order *Lepidoptera,* as described above. Accordingly, this term originally means larvae of various *Psychidae* moths, but when referring to the term "bagworm" with no modifier herein, in principle means *Eumeta japonica* used to construct a cDNA library in the cloning of the rbFib H gene.

As used herein, the term "silk" refers to a thread derived from an insect or an organism of order *Araneae,* which is a proteinous thread spun by a larva or an adult of the insect or the organism of order *Araneae* for the purpose of nest building, migration, anchoring, cocooning, prey capture, and the like. Reference to the term "silk" with no modifier herein in principle means a quite ordinary silk, wherein the origin of the silk is not specified with an organism name. In case of indicating a silk from a particular organism, the name of the organism is placed before the term "silk" as seen in silkworm silk or bagworm silk.

The term "fibroin H chain (Fib H) protein" is one of the proteins constituting fibroin, which is a main fibrous protein component of silk. For example, fibroin of silkworm is mainly composed of three proteins: Fib H protein, Fib L protein, and p25 protein. Among these proteins, Fib H protein is a major constituent protein of fibroin, and the properties of a silk are mainly attributed to the Fib H protein. Accordingly, the Fib H protein, that is a constituent fibrous component of a silk, is often referred to herein as silk. In addition, the origin of Fib H is in principle not specified to a particular organism name by reference to the term "Fib H" with no modifier. In case of indicating Fib H protein from a particular organism, the name of the origin organism or its initial is placed before the term "Fib H", as seen in "silkworm Fib H protein" (sFib H protein) or "bagworm Fib H protein" (bFib H protein).

As used herein, the term "recombinant fibroin H chain protein" (rFib H protein) refers to a Fib H protein that is encoded by a recombinant fibroin H chain gene cloned using a gene cloning technology. The rFib H protein is not necessarily composed of the same amino acid sequence as the wild-type full length Fib H protein, as long as the rFib H protein comprises the basic components of the Fib H protein. The basic components of the Fib H protein comprise an N-terminal region, a central region, and a C-terminal region. These basic components will be detailed in the chapter "1-3. Constitution". In addition, the rFib H protein may not be a Fib H protein derived from a single organism, and may be a chimera Fib H protein composed of polypeptides derived from two or more organisms. For example, it includes a chimera Fib H protein composed of the Fib H protein of a bagworm and that of a silkworm. In the present specification, in cases where more than half of the amino acids in the whole amino acid sequence constituting the rFib H protein are derived from a particular insect, the initials of the name of the particular insect is preceded by "r" in the "rFib H protein". For example, in cases where 90% or more of the amino acid sequence constituting the rFib H protein is derived from the amino acid sequence of a bagworm Fib H protein, the former protein is referred to as an "rbFib H protein".

As used herein, the term "modified-recombinant fibroin H chain protein" (m-rFib H protein) refers to a protein composed of an amino acid sequence different from the amino acid sequence of the wild-type Fib H protein, in which a part of the amino acid sequence has been artificially modified based on the rFib H protein. For example, the m-rFib H proteins comprise mutant rFib H proteins introduced a deletion, an addition, and/or a substitution of one or more amino acid(s) in the rFib H protein. Specifically, for example, the mutant rFib H protein corresponds to the rFib H protein deleted the N-terminal region and/or the C-terminal region, which is/are the basic component(s) of the Fib H protein and the like.

As used herein, the term "fibroin H chain gene" (Fib H gene) refers to a gene encoding the above-described Fib H protein. Similarly, the Fib H protein, the origin of Fib H gene is herein in principle not limited to a particular organism by reference to the term "Fib H gene" with no modifier. In case of indicating a Fib H gene from a particular organism, the name of the origin organism or its initial is placed before the term "Fib H". For example, a Fib H gene from a bagworm is referred to as a bagworm Fib H gene or a bFib H gene.

As used herein, the term "recombinant fibroin H chain gene" (rFib H gene) refers to a Fib H gene cloned using a gene cloning technology, and encoding the recombinant Fib H protein as described above.

As used herein, the term "modified-recombinant fibroin H chain gene" (m-rFib H gene) refers to a gene encoding the above-mentioned m-rFib H protein. Similarly, the term "modified-recombinant bagworm fibroin H chain gene" (m-rbFib H gene) as used herein refers to a gene encoding the "m-rbFib H protein".

As used herein, the term "recombinant bagworm silk" refers to a bagworm silk comprising an rbFib H protein (including an m-rbFib H protein).

As used herein, the term "expression vector" refers to an expression system comprising a gene in a state that allows for the expression, and can control the expression of the gene.

As used herein, the wording "a state that allows for the expression" means that the gene comprised in a vector is incorporated in the vector so that the gene can be expressed in the host cell. Specifically, it means that the gene comprised is placed under the control of a promoter in the expression vector.

### 1-3. Constitution

### 1-3-1. Constitution of recombinant bagworm fibroin H chain (rbFib H) protein

The rbFib H protein in the present specification comprises as basic components the N-terminal region, central region, and C-terminal region in this order from the N-terminal side. Further, the rbFib H protein may comprise a signal sequence and/or a marker peptide. Each of the regions may be directly linked each other, or may be indirectly linked via any linker sequence. Each of the regions will be specifically described below.

### (N-terminal region)

The "N-terminal region" refers to an amino acid region located at the N-terminal side of the below-mentioned central region in the amino acid sequence constituting the rbFib H protein, as shown in Figure 1. Specifically, the N-terminal region corresponds to: the amino acid sequence shown in SEQ ID NO: 1; an amino acid sequence derived from the amino acid sequence shown in SEQ ID NO: 1 having an addition, a deletion, or a substitution of one or a plurality of amino acid(s); or an amino acid sequence having an amino acid identity of 90% or more, 91% or more, 92% or more, 93% or more, 94% or more, 95% or more, 96% or more, 97% or more, 98% or more, or 99% or more to the amino acid sequence shown in SEQ ID NO: 1. Additionally, in the present specification, the amino acid substitution is preferably a conservative amino acid substitution, because a protein carrying a conservative amino acid substitution can have a structure or properties substantially equal to those of the wild-type protein. Conservative amino acid means the relationship among amino acids classified into the same amino acid group. The following groups are known as the above-described amino acid groups: non-polar amino acid group (glycine, alanine, phenylalanine, valine, leucine, isoleucine, methionine, proline, tryptophan); polar amino acid group (amino acids other than non-polar amino acids); charged amino acid group (acidic amino acids (aspartic acid, glutamic acid) and basic amino acids (arginine, histidine, lysine)); uncharged amino acid group (amino acids other than charged amino acids); aromatic amino acid group (phenylalanine, tryptophan, tyrosine); branched amino acid group (leucine, isoleucine, valine); aliphatic amino acid group (glycine, alanine, leucine, isoleucine, valine); and the like. As used herein, the wording "a plurality of' refers to, for example, 2 to 20, 2 to 15, 2 to 10, 2 to 7, 2 to 5, 2 to 4, or 2 to 3. The term "amino acid identity" refers to the ratio (%) of the number of identical amino acid residues to the number of the amino acid residues of SEQ ID NO: 1, when the two amino acid sequences are aligned with introducing gaps, if necessary, into either amino acid sequence such that the highest degree of match between the two is obtained. The amino acid identity can be calculated using a protein search system, such as BLAST, FASTA, and the like (Karlin, S. et al., 1993, Proc. Natl. Acad. Sci. USA, 90: 5873-5877; Altschul, S. F. et al., 1990, J. Mol. Biol., 215: 403-410; Pearson, W.R. et al., 1988, Proc. Natl. Acad. Sci. USA, 85: 2444-2448).

Meanwhile, a Fib H protein usually comprises a signal sequence in the N-terminal side, but the N-terminal region comprises no signal sequence.

### (Central region)

The "central region" is a region that exhibits the physical properties of a bFib H protein, and is composed of a plurality of linked central repeat units consisting of the same and/or different amino acid sequences. The number of the central repeat units is not limited as long as the number is 3 or more. There is no specific upper limit for the number of the central repeat units. For example, the central region may comprise 10, 20, 30, 40, 50, 60, 70, 80, 90, 100, 150, 200, 250, 300, 350, 400, 450, 500, or more central repeat units.

The term "central repeat unit" (herein often referred to as "CRU") consists of 120 to 170 amino acids in full length per unit, and comprises one alanine cluster and a plurality of glycine/alanine units.

The term "alanine cluster" (herein often referred to as "Ala cluster") is a subunit consisting of consecutive alanine (Ala), and is comprised in the N-terminal side in the CRU. One Ala cluster comprises 15 to 25 alanines, and one glutamic acid or glutamine is comprised in the central portion of the Ala cluster (for example, position 10 from the N-terminal side of the Ala cluster) besides the alanines. Specific example of the Ala cluster in a bFib H protein includes, but not limited to, the amino acid sequence shown in SEQ ID NO: 3 or 4.

The term "glycine/alanine unit (G/A unit)" is a unit consisting of two amino acids composed of glycine (Gly: G) and alanine (Ala: A), and is consisting of glycine-alanine (AG) or alanine-glycine (GA). The most part of the CRU is composed of G/A units, and 30 units or more, 35 units or more, or 40 units or more, or 60 units or less, 55 units or less, or 50 units or less G/A units are comprised per CRU.

Furthermore, the CRU may comprise a non-G/A portion consisting of 5 to 7 amino acids other than glycine and alanine. The amino acid sequence of the non-G/A portion is not limited. For example, a suitable non-G/A portion consists of an amino acid sequence shown in SEQ ID NO: 44 or 45 consisting of serine, valine, and tyrosine, or an amino acid sequence shown in SEQ ID NO: 46.

The amino acid sequence constituting the CRU of the bFib H protein is not limited as long as the amino acid sequence satisfies the requirements for each of the constituents as described above. Specific examples comprise, but not limited to, amino acid sequences shown in SEQ ID NOs: 5 to 13.

Each of the CRU constituting the central region of the bFib H protein are mutually linked directly or via any linker sequence consisting of other 1 to 30 amino acids, 1 to 20 amino acids, or 1 to 10 amino acids.

As mentioned above, the central region is a region that exhibits the physical properties of a bFib H protein. The central region of the rbFib H protein has the same or similar physical properties as those of the central region of the wild-type bFib H protein. Here, the central region of the m-rbFib H protein according to the present invention does not differ in the amino acid sequence from the central region of the rbFib H protein, except that only the number of the CRU is varied from it. Accordingly, the m-rbFib H protein has, in principle, the same or similar physical properties as those of the rbFib H protein.

### (C-terminal region)

The term "C-terminal region" refers to an amino acid region located at the C-terminal side of the above-mentioned central region in the amino acid sequence constituting the rbFib H protein, as shown in Figure 1. Specific examples include: the amino acid sequence shown in SEQ ID NO: 2; an amino acid sequence derived from the amino acid sequence shown in SEQ ID NO: 2 having an addition, a deletion, or a substitution of one or a plurality of amino acid(s); or an amino acid sequence having an amino acid identity of 90% or more to the amino acid sequence shown in SEQ ID NO: 2.

### (Signal peptide)

An rbFib H protein according to the present invention may comprise a signal peptide at the N-terminal side of the N-terminal region, if necessary.

The term "signal peptide" (signal sequence) refers to an extracellular transport signal required in extracellularly secreting a protein biosynthesized with gene expression. After translation, signal peptides are cleaved and removed by signal peptidase before extracellular secretion. Signal peptides comprise positively charged amino acids such as lysine or arginine in the N-terminal side, followed by a sequence of amino acids of high hydrophobicity such as alanine, leucine, valine, isoleucine, or phenylalanine.

A Fib H protein usually comprises an endogenous signal peptide at the N-terminal side therein. The bFib H protein also comprises an endogenous signal peptide at the N-terminal side thereof. Accordingly, in cases where an rbFib H protein according to the present aspect of the present invention comprises a signal peptide, the signal peptide may be either an endogenous signal peptide or an exogenous signal peptide. When a synthesized rbFib H protein is efficiently transferred extracellularly, it is preferred to use the signal peptide from a host cell that expresses a protein for extracellular transfer, but not limited to it. For example, if the m-rbFib H gene expression vector according to the third aspect is introduced into a host cell, the m-rbFib H gene comprised in the gene expression vector preferably encodes a signal peptide derived from the host. More specifically, when the host is *E. coli,* it is sufficient for the m-rbFib H gene to comprise the gene sequence that encodes a signal peptide derived from *E. coli.* For the amino acid sequence of the signal peptide or the base sequence encoding it, the known sequences may be used, but not limited to them. For example, the amino acid sequence of the signal peptide from *E. coli* comprises the amino acid sequence shown in SEQ ID NO: 47. In addition, the base sequence encoding the signal peptide from *E. coli* comprises the base sequence encoding the amino acid sequence shown in SEQ ID NO: 47, specifically, the base sequence shown in SEQ ID NO: 48.

The C-terminal side of the signal peptide may comprise a signal sequence post-insertion sequence that promotes the cleavage and the secretion of the signal peptide, and/or an amino acid sequence comprising a recognition site for a signal peptidase that cleaves the signal peptide from the fusion protein. The amino acid sequence of the signal peptide is not particularly limited, and may usually be in the range of 3 to 60 amino acids.

### (Marker peptide)

An rbFib H protein according to the present invention may comprise a marker peptide, if necessary. The term "marker peptide" refers to a peptide that is co-expressed with a polypeptide of interest (here, corresponding to rbFib H protein), and serves as an indicator for detection or extraction of the polypeptide, without inhibiting or reducing the activity of the polypeptide of interest. The marker peptide is usually constituted so as to be co-expressed with an rbFib H protein in the form of a fusion polypeptide. The marker peptide is generally placed at the N-terminal side and/or the C-terminal side in the rbFib H protein, but not limited to these positions. Examples of marker peptides comprise, but not limited to, peptide tags such as a histidine (His) tag (for example, (His)6 to (His)₁₀), FLAG tag, myc tag, and HA tag, and GFP proteins, etc.

Specific examples of the amino acid sequence of an rbFib H protein comprise the amino acid sequence shown in SEQ ID NO: 15 constituting the bw592 protein, or the amino acid sequence shown in SEQ ID NO: 23 constituting the bw753 protein, both proteins are constructed in the Examples as mentioned below. However, the bw592 protein and the bw753 protein comprise a signal peptide derived from *E. coli* consisting of the amino acid sequence shown in SEQ ID NO: 47 at the N-terminus, and a His tag at the C-terminus, as shown in Figure 1.

### 1-3-2. Constitution of modified-recombinant bagworm fibroin H chain (m-rbFib H) protein

An m-rbFib H protein of the present aspect has the constitution of the above-mentioned recombinant bagworm fibroin H chain protein in which all or part of the N-terminal region and/or all or part of the C-terminal region is/are deleted. The term "part" as used herein refers to one amino acid or two or more consecutive or inconsecutive amino acids in the amino acid sequence constituting the N-terminal region or the C-terminal region, wherein the number of amino acid(s) is less than all numbers of the amino acids in the sequence. Preferably, the number of the amino acids is such that the m-rbFib H protein can lose the function of the N-terminal region or the C-terminal region. For example, the part of the amino acids comprises 5, 8, 10, 12, 15, 18, 20, or more consecutive or inconsecutive amino acids.

However, the m-rbFib H protein that can achieve the effects of the present invention is only the protein comprising a constitution in which all or part of the N-terminal region or all or part of the C-terminal region is/are deleted. The protein having a constitution in which all or part of the N-terminal region and all or part of the C-terminal region are deleted is included in the m-rbFib H protein, it cannot achieve the effects thereof, so that the latter protein does not fall under the m-rbFib H protein of interest of the present invention. Accordingly, unless otherwise specified, the m-rbFib H protein as used herein means the rbFib H protein in which all or part of the N-terminal region or all or part of the C-terminal region is/are deleted.

The m-rbFib H protein may comprise a mutation(s), i.e., addition, deletion, and/or substitution of other one or more amino acids to the extent that the function of the central region will not be lost, besides the above-mentioned deletion of the N-terminal region or the C-terminal region. Even in the case where all or part of the N-terminal region is/are deleted, the m-rbFib H protein may have the above-mentioned signal sequence at the N-terminal side.

Specific examples of the amino acid sequence of the m-rbFib H protein include: the amino acid sequence as shown in SEQ ID NO: 16 that constitutes the bw592ΔN protein; the amino acid sequence as shown in SEQ ID NO: 19 that constitutes the bw592ΔC protein; the amino acid sequence as shown in SEQ ID NO: 25 that constitutes the bw753ΔN protein; or the amino acid sequence as shown in SEQ ID NO: 27 that constitutes the bw753ΔC protein; all the proteins are constructed in the Examples as mentioned below. As mentioned above, the amino acid sequence as shown in SEQ ID NO: 21 that constitutes the bw592ΔN/C protein and the amino acid sequence as shown in SEQ ID NO: 29 that constitutes the bw753ΔN/C protein are m-rbFib H proteins. However, they do not achieve the effects of the present invention, so that they are not the m-rbFib H protein according to the present invention.

### 2. Modified-recombinant bagworm fibroin H chain gene (m-rbFib H gene)

### 2-1. Overview

The second aspect of the present invention is an m-rbFib H gene. The m-rbFib H gene of the present aspect is a gene encoding the m-rbFib H protein described in the first aspect.

Expression of the m-rbFib H gene according to the present invention in a host cell, such as *E. coli,* make it possible to make the host cells express recombinant proteins not less than 15 times more than the expression of unmodified rbFib H gene.

### 2-2. Constitution

### 2-2-1. Constitution of recombinant bagworm fibroin H chain (rbFib H) gene

The rbFib H gene in the present specification is the rbFib H gene which was newly cloned by the present inventors from a cDNA library prepared from silkglands of a last instar larva of *Eumeta japonica.* A part sequence of the rbFib H gene is disclosed in JP2018-074403A. The present inventors succeeded in cloning of a new rbFib H gene comprising the N-terminal region and the C-terminal region, that had not yet been identified in JP2018-074403A. The rbFib H gene in the present specification is a recombinant gene reconstituted based on the information of the partial base sequence of the rbFib H gene obtained by cloning, so as to comprise the base sequence encoding the N-terminal region, the central region, and the C-terminal region, all of which are basic components of the rbFib H gene.

In the case of expressing the m-rbFib H protein as a secretory protein, it is also sufficient for the rbFib H gene to comprise a signal DNA encoding a signal peptide at the 5' end of the rbFib H gene because the signal peptide is comprised at the N-terminal side of the m-rbFib H protein as mentioned above.

The base sequence of the rbFib H gene in the present specification is not limited, as long as the gene encodes the rbFib H protein described in the first aspect. Specific examples of the base sequence of the rbFib H gene comprise, the base sequence of the bw592 gene as shown in SEQ ID NO: 14, or the base sequence of the bw753 gene as shown in SEQ ID NO: 22, both of the genes are constructed in the Examples as mentioned below.

### 2-2-2. Constitution of a modified-recombinant bagworm fibroin H chain (m-rbFib H) gene

The m-rbFib H gene in the present aspect is a gene encoding the m-rbFib H protein. The m-rbFib H gene in the present specification is not limited base sequence, as long as the gene encodes the m-rbFib H protein in the first aspect. Specific examples of the base sequence of the m-rbFib H gene include, the base sequence of the bw592ΔN gene as shown in SEQ ID NO: 16, the base sequence of the bw592ΔC gene as shown in SEQ ID NO: 18, the base sequence of the bw753ΔN gene as shown in SEQ ID NO: 24, or the base sequence of the bw753ΔC gene as shown in SEQ ID NO: 26, all of the genes are constructed in the Examples as mentioned below.

### 3. Expression vector of a modified bagworm Fib H chain gene (m-rbFib H gene expression vector)

### 3-1. Overview

The third aspect of the present invention is an expression vector of the m-rbFib H gene. The expression vector according to the present invention comprises the m-rbFib H gene according to the second aspect in a state that allows for the expression in a host cell such as *E*. *coli.*

Introduction of the expression vector according to the present invention into a host enables the transformant by the vector to produce the m-rbFib H protein 15 times or more compared to that of the transformant by the expression vector of the rbFib H gene.

### 3-2. Constitution

### 3-2-1. Components of the expression vector of a modified bagworm Fib H gene

The expression vector of the m-rbFib H gene according to the present invention is constituted to be able to express the m-rbFib H gene in a host cell. The expression vector of the m-rbFib H gene in the present aspect comprises a core vector, the m-rbFib H gene, and a promoter, as essential components. In addition, the expression vector of the m-rbFib H gene can also comprise a terminator, an enhancer, a multicloning site, a selection marker(s), a polyadenylation signal, a ribosome binding site, a replication origin and the like, as optional components. Each of the components of the expression vector of the m-rbFib H gene in the present aspect will be specifically illustrated below.

### (1) Core vector

A core vector is a vector constituting the backbone moiety of the expression vector of the m-rbFib H gene of the present aspect. Various vectors can be used as core vectors. For example, core vectors include: vectors which are capable of autonomous replication, such as plasmids or bacmids; viral vectors; or vectors which are capable of chromosomal integration via homologous recombination. Usually, a plasmid will be sufficient as a core vector. In general, a vector that is replicable in a cell of the host organism into which the core vector is introduced will be selected as a core vector. In addition, the core vector may be a shuttle vector that is replicable between other bacteria such as *E. coli* and an arthropod. Furthermore, protein expression vectors such as pET systems are commercially available from various life science manufacturers, as core vectors in which the below-mentioned promoter, terminator, multicloning site and selection marker, etc. have been already inserted, and those protein expression vectors can be used as an expression vectors of the m-rbFib H gene.

### (2) m-rbFib H gene

The m-rbFib H gene comprised in the expression vectors of the m-rbFib H gene according to the present invention is the m-rbFib H gene described in the second aspect. Thus, an explanation of the gene is omitted here.

The m-rbFib H gene is placed under the control of the below-mentioned promoter in the expression vector of the m-rbFib H gene.

### (3) Promoter

The promoter comprised in the expression vector of the m-rbFib H gene is an essential component that controls the expression of the m-rbFib H gene in the expression vector of the m-rbFib H gene according to the present invention. The promoter is not limited as long as such a promoter can achieve a transcription control function in a host cell. Any promoter known in the art may be used. In general, for example, known promoters include: overexpression promoters that can express a gene of interest excessively in a host; constitutively active promoters that can constantly express gene(s); expression-inducing promoters that can control the expression of gene(s) freely; and the like. Any of the promoters may be used, and such a promoter may be appropriately selected considering the use of the expression vector of the m-rbFib H gene according to the present invention.

### (4) Terminator

The terminator is composed of a base sequence capable of terminating the transcription of the m-rbFib H gene when the gene is expressed in the expression vector of the m-rbFib H gene according to the present invention. The terminator is not limited as long as the sequence can terminate the transcription of the m-rbFib H gene transcribed by a promoter.

### (5) Enhancer

The enhancer is a regulatory factor that controls a promoter, and is an optional component in the expression vector of the m-rbFib H gene according to the present invention. The enhancer may be placed at a position at which the enhancer can control the promoter in the expression vector of the m-rbFib H gene.

### (6) Multicloning site

The multicloning site is a cluster region comprising many restriction sites for cloning, and is an optional component in the expression vector of the m-rbFib H gene according to the present invention. There is no particular limitation on the base sequence constituting the multicloning site or the kind or number of the restriction sites to be comprised in the multicloning site. In addition, the multicloning site in the expression vector of the m-rbFib H gene is not limited to any number or any position to be placed, and is preferably placed at least within the range of the control region of a promoter in the expression vector of the m-rbFib H gene in a state such that the m-rbFib H gene can be expressed.

### (7) Selection marker

The selection marker can function as a marker for confirming that a host has the expression vector of the m-rbFib H gene according to the present invention. In general, a gene encoding an enzyme, a fluorescent protein, a pigment synthesis protein, a luminescent protein, etc. is used as a selection marker. For example, selection markers include: drug-resistant genes (for example, tetracycline-resistant genes, ampicillin-resistant genes, kanamycin-resistant genes, hygromycin-resistant genes, spectinomycin-resistant genes, chloramphenicol-resistant genes, and neomycin-resistant genes); nutrient genes (for example, biosynthetic genes of leucine, uracil, adenine, histidine, lysine, or tryptophan), fluorescent or luminescent reporter genes (for example, luciferase, β-galactosidase, β-glucuronidase (GUS), and GFP), enzyme genes (neomycin phosphotransferase II (NPT II), dihydrofolate reductase, etc.); and enzyme genes such as blasticidin S-resistant genes. One expression vector of the m-rbFib H gene can comprise a plurality of the same or different selection markers.

In the transformant or the progeny thereof according to the third aspect, the "selection marker" can function as a marker for confirming that the host has the expression vector of the m-rbFib H gene according to the present aspect.

### 3-3. Construction of the expression vector of the m-rbFib H gene

An expression vector of an m-rbFib H gene can be constructed using a gene recombination technology known in the art. Without limitation, a general method is that both termini of an m-rbFib H gene obtained are cleaved using a suitable restriction enzyme(s), then purified, and inserted into a restriction site of a core vector that can be linked with the restriction site of the gene, under the control of a promoter in the core vector.

### 4. Transformant or progeny thereof

### 4-1. Overview

The fourth aspect of the present invention is a transformant obtained by introduction of an expression vector of an m-rbFib H gene, or is a progeny of the transformant. The transformant or progeny thereof according to the present invention is characterized by comprising intracellularly the expression vector of the m-rbFib H gene according to the third aspect.

The transformant or progeny thereof according to the present invention makes it possible to mass-produce an m-rbFib H protein having the physical properties as the same or similar to those of the rbFib H protein. Accordingly, such a transformant or progeny thereof can be used as a mass-production system of the rbFib H protein.

### 4-2. Constitution

A transformant according to the present invention comprises intracellularly the expression vector of the m-rbFib H gene according to the third aspect. That is, the host transformed by the introduction of the expression vector of the m-rbFib H gene according to the third aspect is the transformant according to the present aspect. Accordingly, the transformant according to the present aspect is constituted by the host and the expression vector of the m-rbFib H gene according to the third aspect.

In addition, the "progeny thereof' is a progeny of the transformant, and means an offspring individual of the transformant.

Each constituent will be specifically illustrated below.

### (1) Host

A host constituting the transformant according to the present invention is not limited as long as the host is a microorganism or cell that can express the m-rbFib H gene comprised in an expression vector of an m-rbFib H gene. In case where the host is microorganism, it includes, for example, prokaryotic organisms such *Escherichia coli* and *Bacillus subtilis*; and fungi such as *Saccharomyces cerevisiae* and *Schizosaccharomyces pombe.* In addition, in case where the host is eukaryotic cell, it includes culture cells such as insect cells (for example, Sf9, Sf21, SF⁺, High-Five, and BmN4).

### (2) Expression vector of the m-rbFib H gene

An expression vector of the m-rbFib H gene comprised in the transformant or the progeny thereof according to the present invention is the expression vector of the m-rbFib H gene according to the third aspect. The expression vector of the m-rbFib H gene may be integrated into a host genome.

### (3) Progeny

As used herein, the term "progeny" refers to an offspring individual. Offspring individuals comprise both of the asexual individuals which develop through dividing individuals and the like; and gonozooids which develop through sexual reproduction.

In the present aspect, the term "progeny of a transformant" refers to the individual having intracellularly the expression vector of the m-rbFib H gene according to the third aspect among the offspring individuals of the transformant. The progeny is not limited to any generation number as long as the progeny retains the expression vector of the m-rbFib H gene according to the third aspect.

### 4-3. Method of producing a transformant

A transformant according to the present invention is produced by introducing the expression vector of the m-rbFib H gene into a host. The method of introduction of the expression vector of the m-rbFib H gene may be any known introduction method, depending on the host used the introduction of the vector. Examples of the methods of introducing the vector into the bacterium or the yeast include a heat shock method, a calcium ion method, an electroporation method, a spheroplast method, a lithium acetate method, and the like. Any of these technologies is known in the art, and described in various literatures. For example, reference can be made to the methods described in Green & Sambrook, Molecular Cloning: A Laboratory Manual, 4th Ed., (2012), Cold Spring Harbor Laboratory Press. In addition, methods of introducing the vector into a cell, such as, a Lipofectin method (PNAS, 1989, 86: 6077; PNAS, 1987, 84: 7413), an electroporation method, a calcium phosphate method (Virology, 1973, 52: 456-467), a liposome method, a DEAE-Dextran method, and the like are suitably used.

### 5. Method of producing the modified-recombinant bagworm fibroin H chain protein (m-rbFib H protein)

### 5-1. Overview

The fifth aspect of the present invention is a method of producing the m-rbFib H protein. The m-rbFib H protein obtained by the present production method is the protein of interest in the bagworm silk industry. The production method according to the present aspect makes it possible to mass-produce the m-rbFib H protein in the transformant cell such as *E*. *coli.*

### 5-2. Method

A production method according to the present aspect comprises a culture process and a preparation process as essential processes. Each of the processes will be described below.

### (1) Culture process

The "culture process" refers to a process of culturing the transformant or the progeny thereof according to the fourth aspect under the predetermined conditions. The predetermined conditions refer to an optimal culture condition for a host of a transformant. For example, when the host is *E. coli,* such conditions refer to the conditions under which inoculation in a known culture medium such as an LB culture medium is followed by culture under aeration at 37°C for 6 hours to 12 hours. Such culture conditions may be appropriately applied in the known method as the optimal culture method, depending on the kind of the host.

The present process may comprise an expression-inducing step. The "expression-inducing step" is a step of inducing the expression of the m-rbFib H gene inserted in expression vector of the m-rbFib H gene. Whether or not the step is required is determined depending on the kind of a promoter that controls the expression of the m-rbFib H gene in an expression vector of the m-rbFib H gene. For example, in the case where the promoter is an expression-inducing type promoter, this step will be required. On the other hand, in the case where the promoter is a constitutively active type promoter, this step is not required because the m-rbFib H gene is constantly expressed in a transformant. The expression-inducing method may be performed in accordance with the nature of the expression-inducing promoter. For example, in the case where the m-rbFib H gene is placed under the pET system using a T7 RNA polymerase and a T7 promoter in an expression vector of the m-rbFib H gene, addition of an expression-inducing agent such as IPTG to the culture medium activates a lac promoter in the pET system, and the transcription of the inserted gene (here, m-rbFib H gene) placed under the control of the promoter is strongly promoted. Any of these is a known protein expression method, and reference can be made to the Green & Sambrook, 2012 as described above.

### (2) Preparation process

The "preparation process" is a preparation process of preparing an m-rbFib H protein from the culture solution and/or the transformant after the culture process. In the case where the m-rbFib H gene comprised in the vector comprises a signal DNA, the m-rbFib H protein expressed is secreted extracellularly. Thus, many of the m-rbFib H proteins biosynthesized are present in the culture solution. On the other hand, in case where the m-rbFib H gene comprises no signal DNA, the m-rbFib H proteins biosynthesized are accumulated in the host cell. The present process comprises a process of collecting the m-rbFib H protein present in the culture solution or the transformant cell.

A method of collecting the m-rbFib H protein of interest from the culture solution may comprise a protein purification method known in the art. For example, it is possible to obtain a purified m-rbFib H protein, by using a method appropriately selected from an ammonium sulfate precipitation method, ion exchange chromatography, affinity chromatography, gel chromatography and the like, or a combination thereof. In addition, a method of extracting the m-rbFib H protein accumulated in the host cell may be as follows: the host cell is broken through a physical and/or chemical treatment(s), and then, the protein is purified out of the supernatant of the cell solution using the same protein purification method as mentioned above. All of these protein purification methods are known, and reference can be made to Methods in Enzymology, vol. 463: Guide to Protein Purification 2nd ed. (2009).

### Examples

### <Example 1: Construction of recombinant bagworm fibroin H gene>

### (Object)

The object is to newly clone a bagworm Fib H gene and subsequently construct an rbFib H gene on the basis of the information of the base of a bagworm Fib H gene identified in JP2018-074403

### (Method)

Silk glands were isolated from last instar larvae of *Eumeta japonica,* and the total RNA was extracted using RNeasy Mini Kit (from Qiagen N.V.). Subsequently, a cDNA library of *Eumeta japonica* was prepared, using SuperScript III First-Strand Synthesis SuperMix (from Invitrogen) with the total RNA as a template and with a random hexamer as a primer.

### (1) Cloning of 5'-end region of bFib H gene

For cDNA cloning of the 5'-end side of a bFib H gene, a PCR primer pair consisting of the sequences shown in SEQ ID NOs: 30 and 31 was designed. This primer pair and PrimeSTAR GXL DNA Polymerase (from Takara Bio Inc.) were used to perform PCR in accordance with the standard protocol (98°C for 2 minutes; then [98°C for 10 seconds and then 68°C for 2 minutes] × 30 cycles; then 68°C for 2 minutes; then 4°C) as described in the instructions. The amplification products were separated by agarose gel electrophoresis, and subsequently an approximately 1600-bp band was cut out. A DNA fragment was extracted and purified using Wizard (registered trademark) SV Gel and PCR Clean-Up System (from Promega Corporation). The resulting DNA fragment was inserted into the HincII site of pUC118 vector using Mighty Cloning Reagent Set (Blunt End) (from Takara Bio Inc.) to prepare a plasmid vector "pUC118-bw-N" comprising the 5'-end region of a bFib H gene. Subsequently, the base sequence of the inserted fragment was determined. As a result, the information of the base sequence of the 5'-end region of the bFib H gene consisting of 1608 bp as shown in SEQ ID NO: 32, was revealed. This 5'-end region encodes, the signal sequence, the N-terminal region, and part of the central repeat region in this order from the N-terminal side of the bFib H protein.

Next, in order to remove the coding region from the signal sequence, pUC118-bw-N was used as a template to design a PCR primer pair consisting of the base sequences as shown in SEQ ID NOs: 33 and 34, wherein a restriction enzyme cleavage sequence is added, respectively. This primer pair and PrimeSTAR GXL DNA Polymerase (from Takara Bio Inc.) were used to perform PCR. The amplification product was separated by agarose gel electrophoresis to give an approximately 600-pb short band and an approximately 1-kb long band. Each band was cut out, and then, a DNA fragment was extracted and purified using Wizard (registered trademark) SV Gel and PCR Clean-Up System (from Promega Corporation). Subsequently, DNA Ligation Kit, Mighty Mix (from Takara Bio Inc.) was used to insert the DNA fragments of approximately 600-pb and approximately 1-kb, respectively, into the HincII site of the pUC118 vector. The resulting plasmid vectors were named "pUC118-bw-N-S" and "pUC118-bw-N-L" respectively. The base sequence of the insert in each plasmid vector was confirmed, and the information on the 585-bp base sequence shown in SEQ ID NO: 35 and the information on the 1068-bp base sequence shown in SEQ ID NO: 36 was revealed.

### (2) Cloning of 3 '-end region of bFib H gene

For cDNA cloning of the 3'-end side of the bFib H gene, a PCR primer pair consisting of SEQ ID NOs: 37 and 38 was designed. This primer pair and PrimeSTAR GXL DNA Polymerase (from Takara Bio Inc.) were used to perform PCR in accordance with the standard protocol (98°C for 2 minutes; then [98°C for 10 seconds and then 68°C for 2 minutes] × 30 cycles; then 68°C for 2 minutes; and then 4°C) described in the instructions. The amplification product was separated by agarose gel electrophoresis, and then, an approximately 1200-bp band was cut out. A DNA fragment was extracted and purified using Wizard (registered trademark) SV Gel and PCR Clean-Up System (from Promega Corporation). The resulting DNA fragment was inserted into the HincII site of a pUC118 vector using Mighty Cloning Reagent Set (Blunt End) (from Takara Bio Inc.) to prepare the plasmid vector "pUC118-bw-C" comprising the 3'-end region of the bFib H gene. Subsequently, the base sequence of the insert fragment was determined. As a result, the information of the base sequence of the 3'-end region of the bFib H gene consisting of 1179 bp as shown in SEQ ID NO: 39, was revealed. This 3'-end region encodes a part of the central repeat region and the C-terminal region in this order from the N-terminal side of the bFib H protein.

### (3) Construction of rbFib H gene

(i) In order to construct an rbFib H gene, the pUC118-bw-N-S prepared in (1) was treated with NheI/HindIII. The cleavage product was separated by agarose gel electrophoresis, and then, a 585-bp band derived from the pUC118-bw-N-S was cut out of the gel. A DNA fragment was extracted and purified using Wizard (registered trademark) SV Gel and PCR Clean-Up System (from Promega Corporation). The resulting DNA fragment derived from the pUC118-bw-N-S was named "bw-N-S(N/H)". Next, the pUC118-bw-C prepared in (2) was cleaved with Nhe I/Hind III. The resulting open circular plasmid vector was named a "pUC118-bw-C(N/H)", and this pUC118-bw-C(N/H) was subjected to ligation reaction with the above-mentioned bw-N-S(N/H) to obtain a "pUC118-bw592" cloning vector.
(ii) The pUC118-bw592 was treated with EcoRI/Nhel to obtain a cloning vector "pUC118-bw592-C" comprising only the 3'-end side gene. The pUC118-bw-N-L prepared in (1) was cleaved with the same restriction enzyme. A 1068-bp band was cut out of the gel, and a DNA fragment was extracted and purified using Wizard (registered trademark) SV Gel and PCR Clean-Up System (from Promega Corporation). The resulting DNA fragment derived from the pUC118-bw-N-L and corresponding to the 5'-end region of the bFib H gene was named "bw-N-L(E/N)". The pUC118-bw592-C and the bw-N-L(E/N) were subjected to ligation reaction to obtain a "pUC118-bw753" cloning vector. The pUC118-bw592 and the pUC118-bw753 are both rbFib H genes that are different in the number of bases, and that encode the rbFib H proteins derived from *Eumeta japonica* consisting of 592 amino acids and 753 amino acids, respectively. In the present specification, each of the genes is referred to as "bw592 gene" (SEQ ID NO: 14) and "bw753 gene" (SEQ ID NO: 22), respectively. In addition, the rbFib H protein that is encoded by the bw592 gene is referred to as the "bw592 protein", and the amino acid sequence thereof is shown by SEQ ID NO: 15. The bw592 protein consists of the N-terminal region, the three central repeat regions, and the C-terminal region of the bFib H protein, as shown in Figure 1. On the other hand, the rbFib H protein that is encoded by the bw753 gene is referred to as "bw753 protein", and the amino acid sequence thereof is shown by SEQ ID NO: 23. The bw753 protein consists of the N-terminal region, four central repeat regions, and the C-terminal region of the bFib H protein, as shown in Figure 1.

### <Example 2: Construction of the expression vector of the recombinant bagworm fibroin H chain gene, and preparation of E. coli transformant>

### (Object)

The object is to construct an expression vector comprising the rbFib H gene cloned in Example 1 in a state that allows for the expression. In addition, the gene expression vector was introduced in an *E. coli* as the host to prepare the *E. coli* transformant that can express the rbFib H gene.

### (Method)

### (1) Construction of expression vector of rbFib H gene

In order to express the bw592 gene and the bw753 gene prepared in (3) in Example 1 in the *E. coli,* each of the cloning vectors of the pUC118-bw592 and the pUC118-bw753 was treated with NcoI/XhoI to obtain the DNA fragments of bw592 and bw753. Subsequently, expression vectors pET-22b (+) (from Novagen) were treated with NcoI/XhoI in the same manner, and then bw592 and bw753 were inserted into the same respective sites. The resulting expression vectors were named "pET-22b-bw592" and "pET-22b-bw753", respectively.

### (2) Preparation of E. coli transformant that expresses rbFib H gene

Each of the expression vectors of the rbFib H gene constructed in (1) was purified, and then, introduced into a cell of an *E. coli* BL21 (DE3) strain, BLR (DE3) strain, and Rosetta 2 (DE3) strain (all of which are from Novagen), respectively, using a conventional method to prepare an *E. coli* transformant (rbFib H expressing *E. coli* strain) that can express the rbFib H gene.

### <Example 3: Construction of the expression vector of the modified-recombinant bagworm fibroin H chain gene, and preparation of the E. coli transformant>

### (Object)

The object is to construct a modified-rbFib H gene (m-rbFib H gene) in which various mutations are added to the rbFib H gene cloned in Example 1. In addition, the gene expression vector was introduced into *E. coli* as a host to prepare the *E. coli* transformant that can express the m-rbFib H gene.

### (Method)

### (1) Construction of expression vector of m-rbFib HΔC gene

For each of the bw592 gene and the bw753 gene that are rbFib H genes, the ΔC-terminal deletion type variant of the rbFib H gene (referred to as "m-rbFib HΔC gene") which is deleted the base sequences encoding the C-terminal region, but maintained the central repeat region that is highly related to the physical properties of a silk was produced using a PCR method.

PCR were performed using the pET-22b-bw592 and the pET-22b-bw753 constructed in Example 2 as templates using a primer pair (SEQ ID NOs: 40 and 41) having NcoI/XhoI cleavage site at the 5'-end side. The amplification products were separated by agarose gel electrophoresis, and then, a band of a predicted size was cut out. DNA fragments were extracted and purified using Wizard (registered trademark) SV Gel and PCR Clean-Up System (from Promega Corporation). The resulting DNA fragments were treated with NcoI/XhoI to obtain NcoI/XhoI-DNA fragments of the "bw592ΔC" (SEQ ID NO: 18) and the "bw753ΔC" (SEQ ID NO: 26) that are ΔC variants of the bw592 and the bw753, respectively. These DNA fragments were each inserted into the NcoI/XhoI site of the pET-22b (+) (from Novagen) treated with NcoI/XhoI. The resulting expression vectors were named "pET-22b-bw592ΔC" and "pET-22b-bw753ΔC", respectively. In this regard, the rbFib H protein encoded by the bw592ΔC gene is referred to as the "bw592ΔC protein", and the amino acid sequence thereof is shown by SEQ ID NO: 19. In addition, the rbFib H protein encoded by the bw753ΔC gene is referred to as the "bw753ΔC protein", and the amino acid sequence thereof is shown by SEQ ID NO: 27.

### (2) Construction of expression vector of m-rbFib HΔN gene

For each of the bw592 gene and the bw753 gene that are rbFib H genes, the ΔN-terminal deletion type variant of the rbFib H gene (referred to as "m-rbFib HΔN gene") which is deleted the base sequences encoding the N-terminal region, but maintained the central repeat region that is highly related to the physical properties of a silk was produced using a PCR method.

PCR were performed using the pET-22b-bw592 and the pET-22b-bw753 constructed in Example 2 as templates using a primer pair (SEQ ID NOs: 42 and 43) having NcoI/XhoI cleavage site at the 5'-end side. The amplification products were separated by agarose gel electrophoresis, and then, bands of a predicted size were cut out. DNA fragments were extracted and purified using Wizard (registered trademark) SV Gel and PCR Clean-Up System (from Promega Corporation). The resulting DNA fragments were treated with NcoI/XhoI to obtain NcoI/XhoI-DNA fragments of the "bw592ΔN" (SEQ ID NO: 16) and the "bw753ΔN" (SEQ ID NO: 24) that are ΔN variants of the bw592 and the bw753, respectively. These DNA fragments were each inserted into the NcoI/XhoI site of pET-22b (+) (from Novagen) treated with NcoI/XhoI. The resulting expression vectors were named "pET-22b-bw592ΔN" and "pET-22b-bw753ΔN" respectively. In this regard, the rbFib H protein encoded by the bw592ΔN gene is referred to as the "bw592ΔN protein", and the amino acid sequence thereof is shown by SEQ ID NO: 17. In addition, the rbFib H protein encoded by the bw753ΔN gene is referred to as the "bw753ΔN protein", and the amino acid sequence thereof is shown by SEQ ID NO: 25.

### (3) Construction of expression vector of m-rbFib HΔN/C gene

For each of the bw592 gene and the bw753 gene that are rbFib H genes, a both-terminal deletion type variant of the rbFib H gene (referred to as "m-rbFib HΔN/C gene") which is deleted the respective base sequences encoding the N-terminal region and the C-terminal region, but not maintained the central repeat region that is highly related to the physical properties of a silk was produced using a PCR method.

A PCR was performed using the pET-22b-bw592 and the pET-22b-bw753 constructed in Example 2 as templates using a primer pair (SEQ ID NOs: 42 and 41). The amplification product was separated by agarose gel electrophoresis, and then, a band of a predicted size was cut out. A DNA fragment was extracted and purified using Wizard (registered trademark) SV Gel and PCR Clean-Up System (from Promega Corporation). The resulting DNA fragments were treated with NcoI/XhoI to obtain NcoI/XhoI-DNA fragments of the "bw592ΔN/C" (SEQ ID NO: 20) and the "bw753ΔN/C" (SEQ ID NO: 28) that are ΔN/C variants of the bw592 and the bw753, respectively. These DNA fragments were each inserted into the NcoI/XhoI site of pET-22b (+) (from Novagen) treated with NcoI/XhoI. The resulting expression vectors were named "pET-22b-bw592ΔN/C" and "pET-22b-bw753ΔN/C" respectively. In this regard, the rbFib H protein encoded by the bw592ΔN/C gene is referred to as the "bw592ΔN/C protein", and the amino acid sequence thereof is shown by SEQ ID NO: 21. In addition, the rbFib H protein encoded by the bw753ΔN/C gene is referred to as the "bw753ΔN/C protein", and the amino acid sequence thereof is shown by SEQ ID NO: 29.

### (4) Preparation of E. coli transformant that expresses rbFib H gene

Each of the expression vectors of an m-rbFib H gene constructed in (1) to (3) was purified, and then, introduced into a cell of an *E. coli* BL21 (DE3) strain (from Novagen) using a routine method to prepare an *E. coli* transformant (m-rbFib H expression *E. coli* strain) that can express an m-rbFib H gene. The transformants into which the bw592 gene, the bw592ΔN gene, the bw592ΔC gene, the bw592ΔN/C gene, the bw753 gene, the bw753ΔN gene, the bw753ΔC gene, and the bw753ΔN/C gene are introduced, are referred to as a bw592 strain, a bw592ΔN strain, a bw592ΔC strain, a bw592ΔN/C strain, a bw753 strain, a bw753ΔN strain, a bw753ΔC strain, and a bw753ΔN/C strain, respectively.

### <Example 4: Verification of expression level of bFib H protein using E. coli transformant>

### (Object)

The object is to express the rbFib H protein or the m-rbFib H protein using the rbFib H expression *E. coli* strain or the m-rbFib H expression *E. coli* strain, respectively, prepared in Examples 2 and 3 and to verify the expression level thereof.

### (Method)

The rbFib H expression *E. coli* strains (bw592 strain and bw753 strain) or the m-rbFib H expression *E. coli* strains (bw592ΔN strain, bw592ΔC strain, bw592ΔN/C strain, bw753ΔN strain, bw753ΔC strain, and bw753ΔN/C strain) prepared in Examples 2 and 3 were each inoculated in an LB culture medium (from Thermo Fisher Scientific Inc.) and cultured at 37°C, and then the turbidity was measured by using WPA CO8000 Cell Dense Meter (from Biowave). At the time point that the turbidity reached 0.4 to 0.6, IPTG was added in such a manner that the final concentration became 1 mM, and the strains were further cultured at 25°C for 4 hours. After the culture, the strains were harvested by centrifugation, suspended in a buffer for solubilization (BugBuster reagent (from Merck) supplemented with Lysonase (from Merck) and PMSF (from Sigma-Aldrich Co. LLC)), left to stand at room temperature for 10 minutes, and centrifuged at 20,000×G at 4°C for 15 minutes to collect supernatants. The supernatants were electrophoresed with a 10% or 12% SDS-polyacrylamide gel, and then, the peptide separated was transferred onto a PVDF membrane using Trans-Blot Turbo^{™} transfer system (from Bio-Rad Laboratories, Inc.) under application conditions of 1.3A, 25V, and 7 minutes.

In the *E. coli* strains prepared in Examples 2 and 3, both the rbFib H protein and the m-rbFib H protein expressed from the respective expression vectors have a His₆ tag at the C-terminus. Then, the PVDF membrane after the transfer treatment was subjected to blocking treatment with skim milk, and then, was allowed to react in accordance with the attached protocol using a 5,000-fold diluted HRP-labeled anti-His antibody (Anti-His-tag mAb-HRP-DirecT, from Medical & Biological Laboratories Co., Ltd.). Subsequently, a luminescent reaction based on the HRP activity was performed by using a luminescent substrate Western Lightning Plus-ECL (from PerkinElmer, Inc.).

### (Results)

The results are shown in Figure 2. This Figure reveals that the rbFib H protein of interest was expressed in the bw592 strain that expresses an unmodified rbFib H protein, but the expression level was not abundant (Lane 1). In cases where an rbFib H protein comprising the basic components of a Fib H protein is expressed in *E. coli,* it appears that some control mechanism exerts an expression suppression action. This unknown control mechanism is extremely inconvenient for object of the present invention, i.e., the object of mass-producing an rbFib H protein in a microorganism expression system and the like.

On the other hand, in the b/w592ΔN strain that expresses an m-rbFib H-bw592ΔN protein removed the N-terminal region of the Fib H protein, the expression level of the m-rbFib H-bw592ΔN protein was markedly increased, and also the degradation of the m-rbFib H-bw592ΔN protein was not observed (Lane 3). Accordingly, it has been made clear that the rbFib H protein with protease resistance can be mass-produced by using a gene encoding the m-rbFib H-bw592ΔN protein removed all or part of the N-terminal region.

In contrast, in the bw592ΔC strain that expresses the m-rbFib H-bw592ΔC protein removed the C-terminal region of the Fib H protein, unlike the bw592ΔN strain, many degradation products of the m-rbFib H-bw592ΔC protein were confirmed (Lane 2). This result suggests that the C-terminal region contributes to the protease degradation resistance of the rbFib H protein. However, in the bw592ΔC strain, the expression of the m-rbFib H-bw592ΔC protein in an overwhelming level exceeding the degradation level, could be confirmed. Accordingly, it has been made clear that the rbFib H protein can be mass-produced by using a gene which encodes the m-rbFib H-bw592ΔC protein removed all or part of the C-terminal region, and extracting the undegraded products after the expression.

Furthermore, the expression level of the m-rbFib H protein was also verified in the bw592ΔN/C strain that expresses an m-rbFib H-bw592ΔN/C protein maintaining substantially only the central region, but removed both N-terminus region and C-terminus region of the Fib H protein. As a result, the expression level of the m-rbFib H protein in the bw592ΔN/C strain was increased compared to in the bw592 strain that expresses the rbFib H protein, but at the same time, the degradation product level was also increased. On the other hand, the increase in the expression level of the m-rbFib H-bw592ΔN/C protein was not remarkable as in the bw592ΔC strain (Lane 4).

This tendency was the same as in the bw753 strain, bw753ΔN strain, bw753ΔC strain, and bw753ΔN/C strain (not shown).

In view of this, the luminescence intensity of the bw592 strain and the bw592ΔC strain, the bw753 strain and the bw753ΔC strain was quantitated using Lumino Image Analyzer Amersham Imager 600 (from GE Healthcare Inc.), and calculated using a dedicated software (v.1.2) attached to the instrument, and then, the expression level of each of the rbFib H protein and the m-rbFib H protein in each strain was determined. The results are shown in Table 1.

**[Table1]**

| Transformant | Expression Amount (Relative Value) |
|---|---|
| bw592 | 1.0 |
| bw592ΔC | 20.4 |
| bw753 | 1.0 |
| bw592ΔC | 16.2 |

| | |
|---|---|
| (n=4) | |

Table 1 shows the relative expression level of each of the bw592ΔC strain and the bw753ΔC strain, assuming that the expression level of the bw592 strain and the bw753 strain expressed in *E. coli* were each 1. It has been recognized that the expression level was approximately 20 times larger in the bw592ΔC strain than in the bw592 strain, and approximately 16 times larger in the bw753ΔC strain than in the bw753. The only difference between the bw592ΔC strain and the bw753ΔC strain was the number of CRUs.

The above-mentioned results demonstrated that, in cases where the rbFib H protein is expressed in a cell of a microorganism such as *E. coli,* the expression level of an m-rbFib H protein can be enhanced by using a gene encoding the m-rbFib H protein in which all or part of any one of the N-terminal regions or the C-terminal region is/are removed, regardless of the number of CRUs. On the other hand, the results also revealed that, in cases where both of the termini were removed, the expression level was increased compared to the expression level of the rbFib H protein, but the degradation level was also increased, and the relative expression level was decreased.

All publications, patents, and patent applications cited herein should be incorporated herein by reference in their entirety.

## Claims

1. A modified-recombinant bagworm fibroin H chain protein obtained by modifying a recombinant bagworm fibroin H chain protein comprising an N-terminal region, a central region, and a C-terminal region in this order from the N-terminal side,
wherein the modified-recombinant bagworm fibroin H chain protein deleting all or part of the N-terminal region or all or part of the C-terminal region thereof, wherein:
the N-terminal region consists of
an amino acid sequence shown in SEQ ID NO: 1,
an amino acid sequence derived from the amino acid sequence shown in SEQ ID NO: 1 having an addition, a deletion, or a substitution of one or a plurality of amino acid(s), or
an amino acid sequence having an amino acid identity of 90% or more to the amino acid sequence shown in SEQ ID NO: 1,
the central region has three or more linked central repeat units consisting of the same and/or different amino acid sequences; and
the C-terminal region consists of
an amino acid sequence shown in SEQ ID NO: 2,
an amino acid sequence derived from the amino acid sequence shown in SEQ ID NO: 2 having an addition, a deletion, or a substitution of one or a plurality of amino acid(s), or
an amino acid sequence having an amino acid identity of 90% or more to the amino acid sequence shown in SEQ ID NO: 2,
wherein the central repeat unit comprises 30 units or more of 2-amino-acid units consisting of glycine and alanine, comprises an alanine cluster comprising 15 to 25 alanines at the N-terminal side, and consists of 120 to 170 amino acids in full length.

2. The modified-recombinant bagworm fibroin H chain protein according to claim 1, wherein the alanine cluster consists of the amino acid sequence shown in SEQ ID NO: 3 or 4.

3. The modified-recombinant bagworm fibroin H chain protein according to claim 2, wherein the central repeat unit is selected from the amino acid sequences shown in SEQ ID NOs: 5 to 13.

4. A modified-recombinant bagworm fibroin H chain gene encoding the modified-recombinant bagworm fibroin H chain protein according to any one of claims 1 to 3.

5. The modified-recombinant bagworm fibroin H chain gene according to claim 4, consisting of any one of the base sequences shown in SEQ ID NO: 16, 18, 24, or 26.

6. An expression vector of a modified-recombinant bagworm fibroin H chain gene, comprising the modified-recombinant bagworm fibroin H chain gene according to claim 4 or 5 in a state that allows for the expression in a host cell.

7. A transformant consisting of a host comprising the expression vector of the modified-recombinant bagworm fibroin H chain gene according to claim 6, or a progeny thereof.

8. The transformant or the progeny thereof according to claim 7, wherein the host is a microorganism or an insect culture cell.

9. A method of producing a modified bagworm fibroin H chain protein, comprising:
a culture process of culturing the transformant or the progeny thereof according to claim 7 or 8 under predetermined conditions; and
a preparation process of preparing the modified bagworm fibroin H chain protein from the culture solution and/or the transformant after the culture process.
